# EUROPEAN PATENT APPLICATION

(11) **EP 2 990 018 A1**
(43) Date of publication of application: **02.03.2016**
(21) Application number: 14002961.2
(22) Date of filing: 27.08.2014
(51) Int. Cl.: A61F 13/49, A61F 13/514, A61F 13/56, A61F 13/15, B32B 5/02, B32B 5/04, B32B 3/26, D04H 13/00

(54) **Stretchable laminate**

(71) Applicant: Hayat Kimya Sanayi Anonim Sirketi, 34662 Altunizade, Uskudar/Istanbul (TR)
(72) Inventor: Guler, Ibrahim, 41275 Basiskele/KOCAELI (TR); Koc, Fikret, 41275 Basiskele/Kocaeli (TR); Erguney, Fatih, 41275 Basiskele/Kocaeli (TR); Uzun, Nimet, Basiskele/Kocaeli (TR); Soylemez, Serdar, 41275 Basiskele/Kocaeli (TR)

(57) **Abstract**

The present invention relates to a stretchable laminate. In particular, this invention relates to the use of the stretchable laminate in absorbent hygienic articles. Such hygienic articles have improved elasticity, fastening properties and better fit resulting in improved anti-leakage properties due to adjustable elastic properties of the stretchable laminate. Elasticity is adjusted by forming recesses of a definite distribution on a fabric layer without applying any elongation pre-forces to the layer and deforming it. Additionally, changing the distribution of the recessed parts provides variability of elasticity in a wide range for a wide range of materials which the fabric layer is produced from in a simple and economical manner. This way enables to obtain stretchable laminates of different elasticity by easily changing the distribution of the recessed parts without changing the dimensions and other basic physical characteristics of the fabric layer. Since the design parameters of the stretchable laminate are so diversified, preparation of the stretchable laminates ,which have varying elastic properties , by using fixed amount/size of the fabric layer is accomplished, whereby still shape, appearance and aesthetics of the laminate is maintained.

## Description

### FIELD OF THE INVENTION

This application relates to stretchable laminates. Particularly, this application relates to the use of the stretchable laminates in absorbent hygienic articles where elasticity is required and/or elasticity can be an additional benefit. To the present invention related stretchable laminates of absorbent hygienic articles can be used in baby diapers, adult diapers, training pants, pull-up garments, feminine sanitary napkins and the like.

### BACKGROUND OF THE INVENTION

Absorbent hygienic articles are indispensable consumer goods which are designed to provide the comfort of human body. The most important function of an absorbent article is prevention of wearer's body from leakage of bodily exudates. Among the other components fastening elements are the ones which are the most effective in providing anti-leakage securing and the fit of the absorbent article to the wearer's body. A fastening system generally consists of extensible elements and fixing elements which join forces in order to encircle the wearer's body for an appropriate fit.

Extensible elements are placed among others on the back region of an absorbent article as rear side panels. Extensible elements are generally elastic laminates which comprise an elastic layer and one or more fabric layer(s). Fabric layer is generally inelastic material in nature and consists of a web-like material such as nonwoven. It is pretreated in order to activate it to behave like an elastic material in lamination process. Therefore, it can be inferred that activation method of fabric layer defines the properties of the laminate. Conventionally, stretching, ring rolling and/or tension-induced tearing is often used for activation to date.

EP0575509 Buel K et al patent is related to imparting elasticity to zero strain stretch laminate using multiple rolling assembly by non-incremental stretching force application to the web.

EP0714351 Wu Pai et al patent is related to laminated sheet comprising elastomeric film and nonwowen fibrous web prepared by stretching with rollers which impart a recoverable elasticity to the laminate film.

EP0217032 patent to Draper Taylor et. al. represents an activation method wherein unstretched nonwoven web is adhered to a stretched elastic film.

Mechanical forces described above for activation cause deformation in material structure resulting in regional deficiencies and weak areas can appear toward the stretch direction of material. Tensile strength and retraction properties can be reduced due to material fatigue caused by pre-elongation forces. In traditional mechanical activation methods, fabric surfaces are in contact with machinery equipment for prolonged time which increases time of exposure of fabric surfaces to friction forces. This may cause linting formation on fabric surfaces which may cause skin irritation. All the effects of traditional mechanical activation methods on fabric layers mentioned above result in reduction of intermediate product or end product quality and/or stability. In addition, the steps of ring rolling process are complicated, time consuming and have low repeatability.

Therefore, there is still existing need for a cost effective activation of webs to be used in elastic laminates of predetermined quality in a simple and economical manner without complicated steps.

Additionally, activation methods based on application of tension forces require the use of nonwoven materials as a fabric layer having special characteristics such as excess stretchability in machine direction. Nonwoven materials are generally stretchable in this direction so as to be run in machinery. But they should be much more stretchable to resist to the tension forces applied in activation without experiencing any damage or tearing. Nonwovens having stiff bonded structures like spunbond nonwovens cannot fulfill the requirements mentioned above. They are not suitable for tension based activation as the activation may ruin the material structure. Nonwovens having loose bonded structures like thermobond or spunlace nonwovens are more preferable for tension based activation as they are more tolerant to the tension forces and can expand without tearing. As a result, material selection is a critical step and has many constraints which hinder applicability of the tension based activation methods for a wide range of nonwoven materials. In the present invention the activation method to be used will not be based on application of tension forces. Therefore, the present invention eliminates the drawbacks about the material selection mentioned above providing use of all types of nonwoven materials.

Furthermore, elasticity can be sometimes required in other regions of the diapers rather than the elastic rear side panels which may provide an additional benefit. Elastic outer covers can be more preferable in some cases especially in pull-up pants. In addition, components having flexible characteristics can be required in order to make the absorbent articles less stiff and thereby more comfortable by reducing feel of contact of a foreign material to skin of wearer. For example, wings of a sanitary napkin can cause a feel of discomfort if they have stiff structures and do not fit to wearer's body and movements. These drawbacks can be eliminated by using elastic materials for said components to make them more flexible and adapted to wearer's body and movements. Elastic laminates can alter the characteristics of the components changing in use properties of them and absorbent articles including them in a positive manner. Therefore, use of stretchable laminates for such components provides improvement in overall performance of the component and absorbent articles including them.

The present invention discloses a new perspective of obtaining a stretchable laminate and eliminates the drawbacks of traditional mechanical activation methods mentioned above.

### DRAWINGS

Figures 1A and 1B plan views and Figures 1C-1F are cross-sectional views of a stretchable laminate comprising an elastic layer and a fabric layer, the stretchable laminate is in a relaxed or retracted state in Figures 1A-1D and in an elongated state in Figures 1E and 1F.
Figures 2A-2D are plan views and Figures 2E-2L are cross-sectional views of a stretchable laminate comprising an elastic layer and two fabric layers, the stretchable laminate is in a relaxed or retracted state in Figures 2A-2F, 2I and 2J and in an elongated state in Figures 2G, 2H, 2K and 2L.
Figures 3A-3Z are cross-sectional views of some possible designs for size and location of activated zones of a stretchable laminate comprising an elastic layer and two fabric layers, both the first and second fabric layers are generally activated (Figures 3B-3Z), the elastic layer and fabric layers have the same width.
Figures 4A-4Z are cross-sectional views of some possible designs for size and location of activated zones of a stretchable laminate comprising an elastic layer and two fabric layers, both the first and second fabric layers are activated, the width of the elastic layer is smaller than the widths of the fabric layers and extends continuously through the width of the activated zone.
Figures 5A-5G are cross-sectional views of some possible designs for size and location of activated zones of a stretchable laminate comprising an elastic layer and two fabric layers, both the first and second fabric layers are activated, the width of the elastic layer is smaller than the widths of the fabric layers and extends discontinuously through the width of the activated zone.
Figure 6 is an aperture forming embodiment which is used to make apertures on the first fabric layer and/or the second fabric layer of the stretchable laminate.
Figure 7A is a schematic view and Figure 7F is an example of illustration of manufacturing method of the stretchable laminate and Figures 7B-7E schematic views of some possible methods for lamination of the fabric layer(s) to the elastic layer.
Figures 8A-8D are plan views of side panels comprising the elastic laminate and Figures 8E-8H are plan views of absorbent articles comprising the side panels, the side panels are placed on both on the right and left hand side of the absorbent articles.
Figures 9A-9B are plan views of fastening tapes comprising the elastic laminate and 9C-9E are plan views of absorbent articles comprising the fastening tapes, fastening tapes are placed on both on the right and left hand side of the absorbent articles.

### DESCRIPTION OF DISCLOSURE

Before going through the details of the invention some related key terms are explained in order to make the rest of the text more understandable.

The term "machine direction" refers to a direction which a material runs along it in machinery.

The term "cross direction" refers to a direction which is perpendicular to the machine direction.

The term "stretch" refers to elongation of a material in a direction when a force is applied in this direction.

The term "primary direction of stretch" refers to a direction in which a material exhibits its maximum stretchability. Materials are generally more stretchable in one direction than the other one.

The term "axis of stretch" refers to an axis to which a force is applied to stretch the material.

The term "elastic" refers to a property of a material which elongates by many times higher of its original size in the primary direction of stretch upon application of a force and return almost to its initial position back after the applied force is removed.

The term "fabric layer" refers to a layer which is produced from fibers.

The term "laminate" refers to a single layer which is produced by combining two or more layers with a definite bonding method.

The term "activation" refers to a definite method of making an inelastic material to gain ability of stretch.

The term "activated" refers to a state for which activation is applied.

The term "activated zone" refers to a zone for which activation is applied to.

The term "non-activated" refers to a state for which activation is not applied.

The term "non-activated zone" refers to a zone for which activation is not applied to.

The term "functional zone" refers to a zone which is used for a definite purpose in production and/or in use.

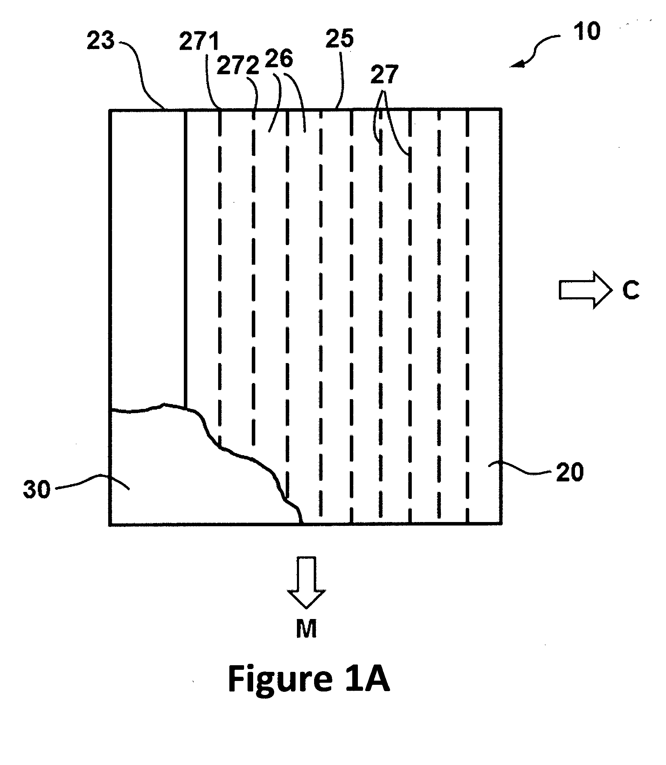

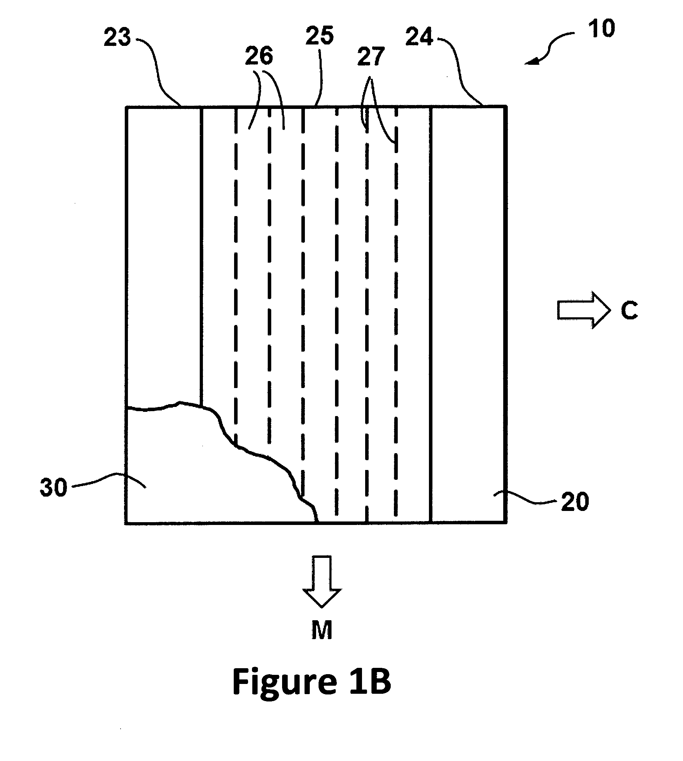

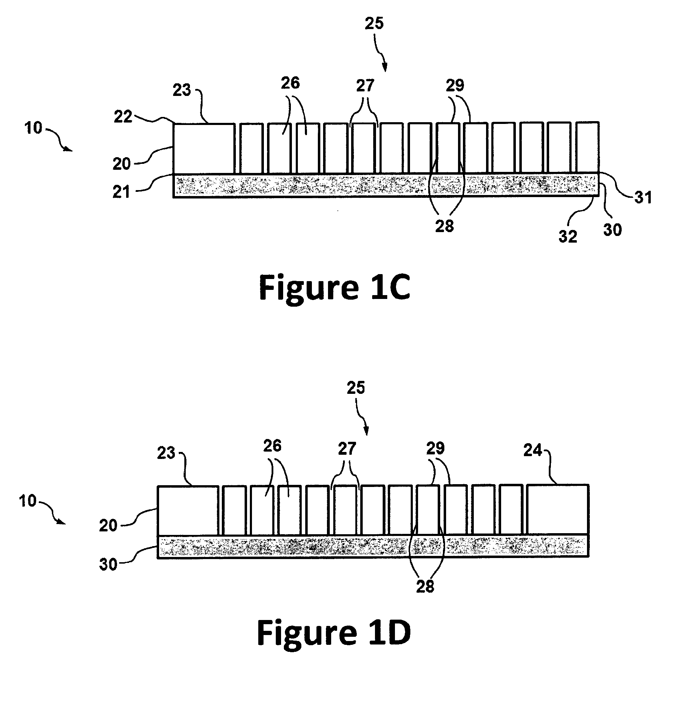

An elastic laminate (10) of the present invention is shown in Figures 1A - 1F. The elastic laminate (10) comprises a fabric layer (20) and an elastic layer (30). The fabric layer (20) has a first surface (21) and a second surface (22). The elastic layer (30) has a first surface (31) which is in contact with the first surface (21) of the fabric layer (20) and a second surface (32). The fabric layer (20) comprises at least one non-activated zone called first non-activated zone (23) and preferably second non-activated zone (24) and at least one activated zone (25). Existence of at least one functional zone (23) is essential both during and after activation to sustain the integrity of the fabric layer (20) and to prevent it from splitting apart.

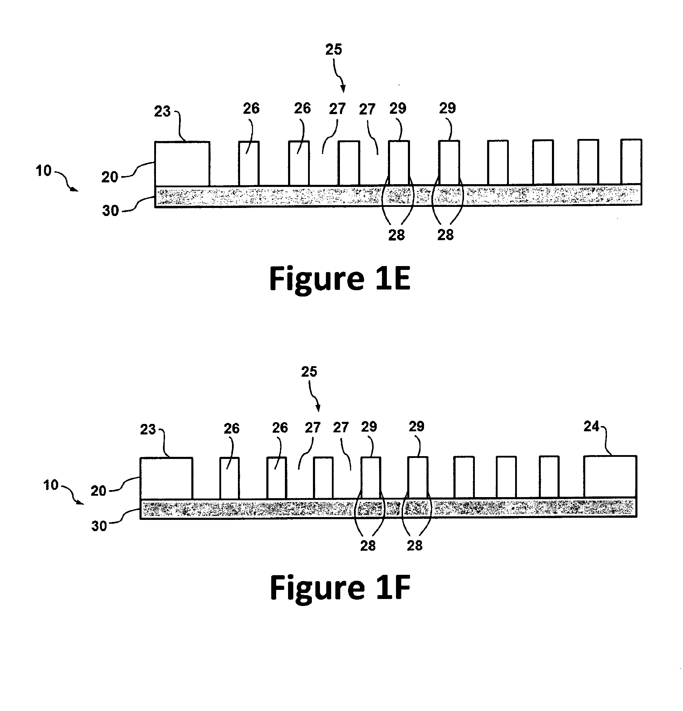

The non-activated fabric zones (23, 24) can be regarded as open areas which are intentionally left blank for further processing steps and/or a force application area for a wearer, mother or caregiver. Therefore later on in the text "functional zone" term will be used instead of the "non-activated zone" term. The activated zone (25) of the fabric layer (20) is cut into several pieces (26) forming apertures (27) which extends through the fabric thickness. The apertures (27) follow a definite order both in the cross (C) and machine (M) direction. The activated zone (25) consists of several apertured lines (271, 272) along the cross direction (C) wherein the apertures (27) extend intermittently along the said lines (271, 272) in the machine direction (M). The apertures (27) in the first line (271) are not aligned to the apertures (27) in the second line (272). The rest of the couples of the lines follow the same order with the couple of the first line (271) and second line (272). The rows are oriented in brick line pattern. Each fabric piece (26) obtained by formation of the apertures (27) has two recessed sides (28) close to the elastic layer (30) and a protruding side (29) far from the elastic layer (30). The fabric pieces (26) are close to each other when the laminate (10) is in a relaxed or retracted state (Figures 1A-1D). They separate from each other in the cross direction (C) when the laminate (10) is elongated (Figures 1E and 1F).

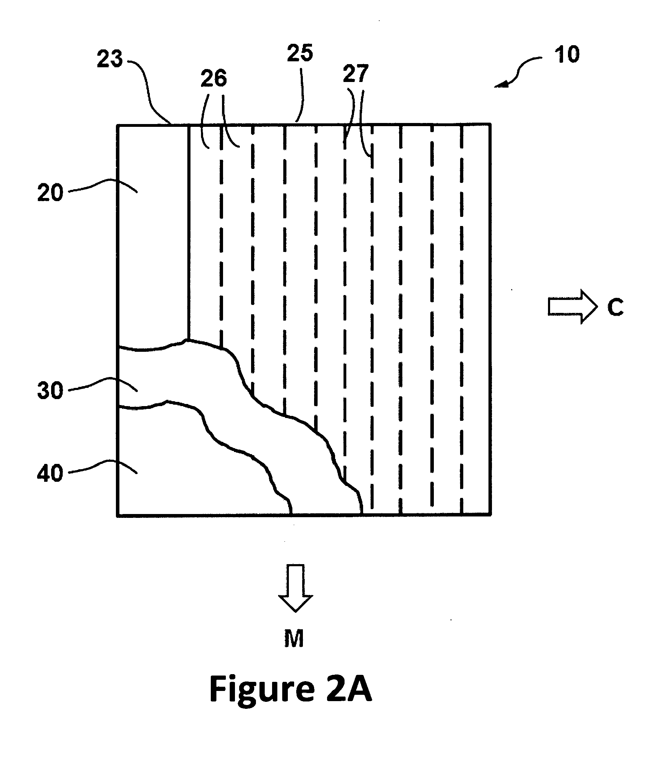

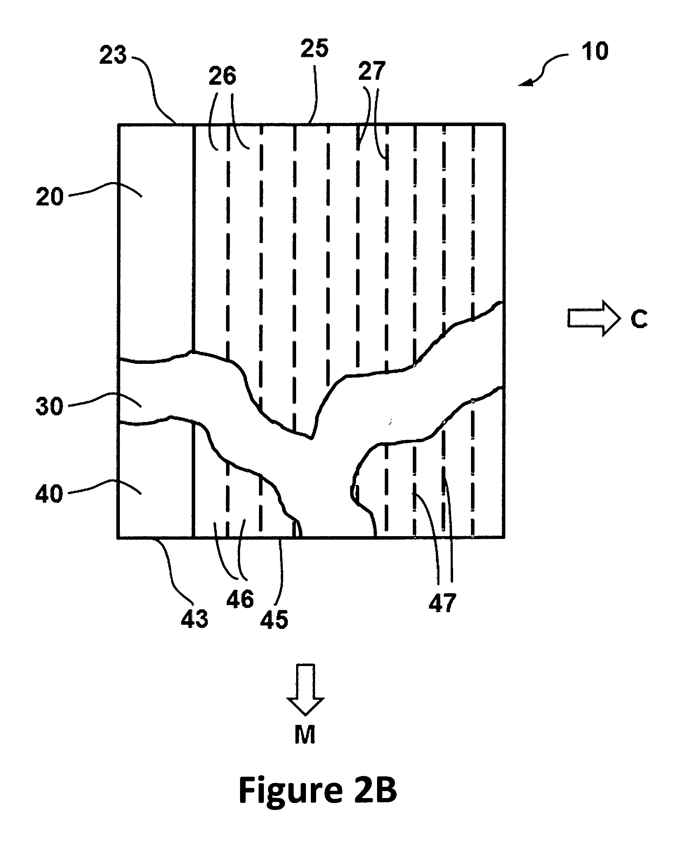

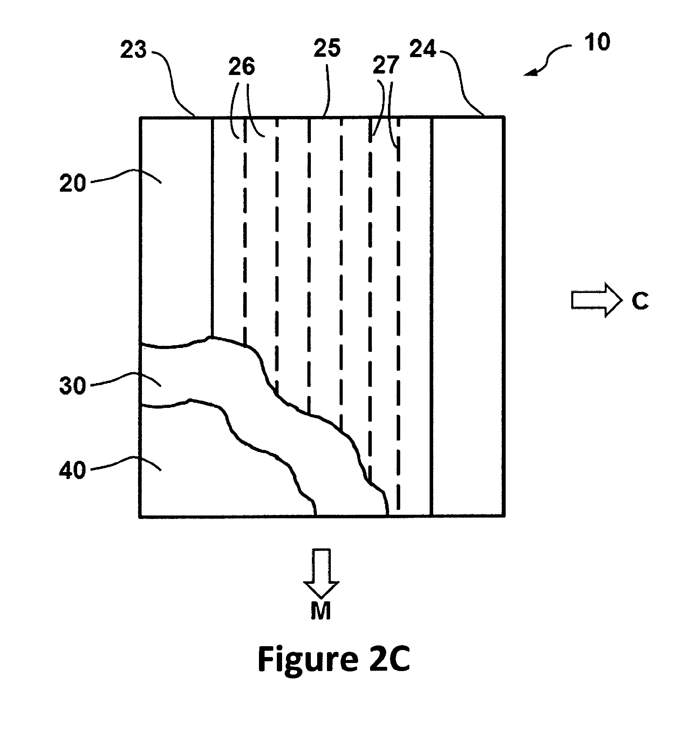

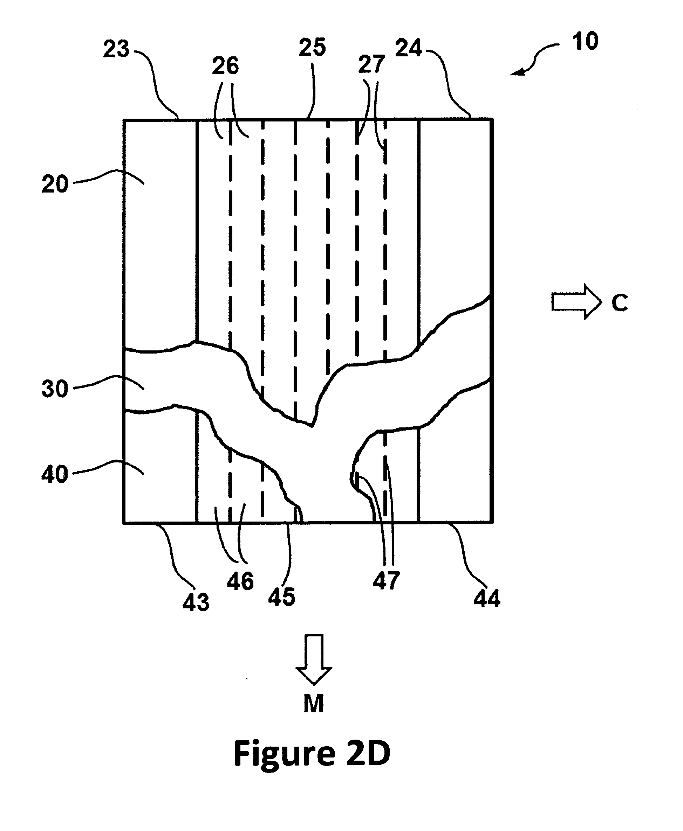

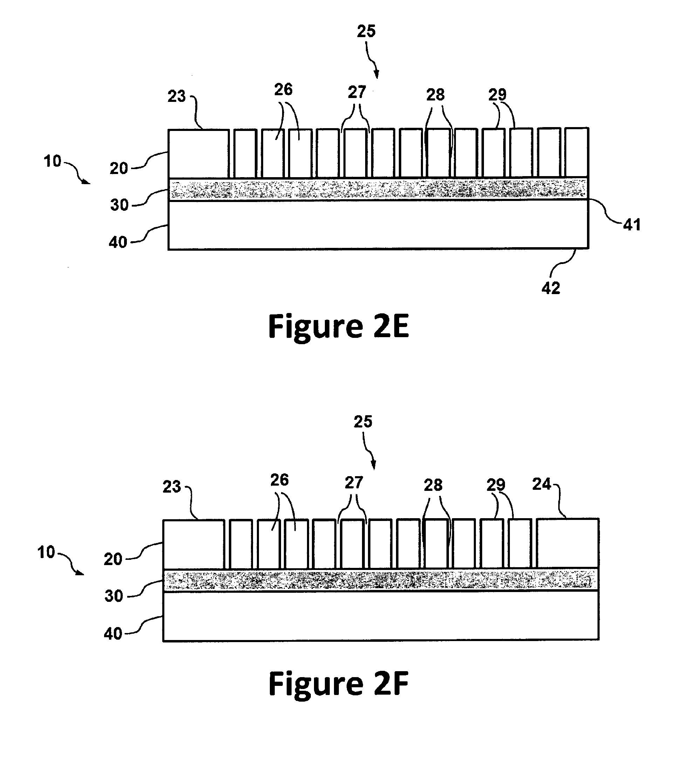

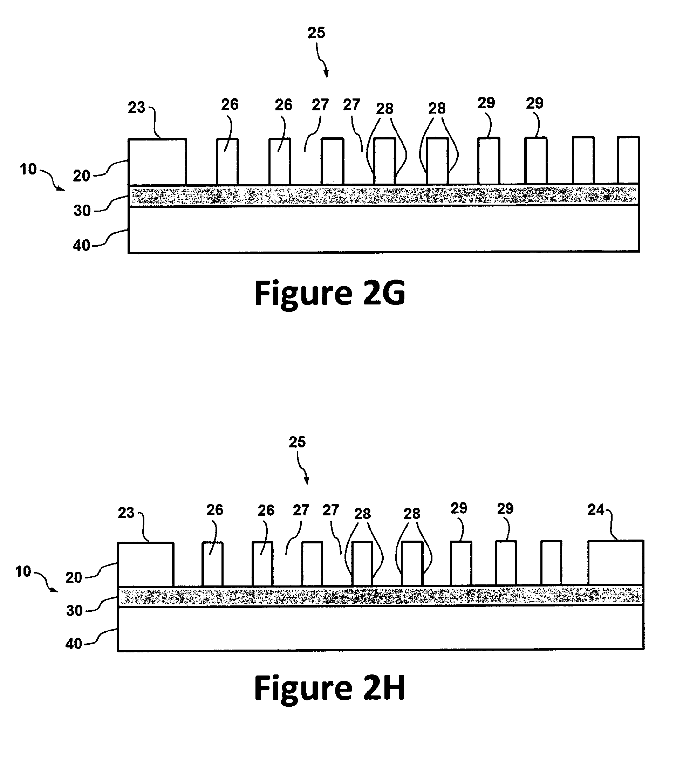

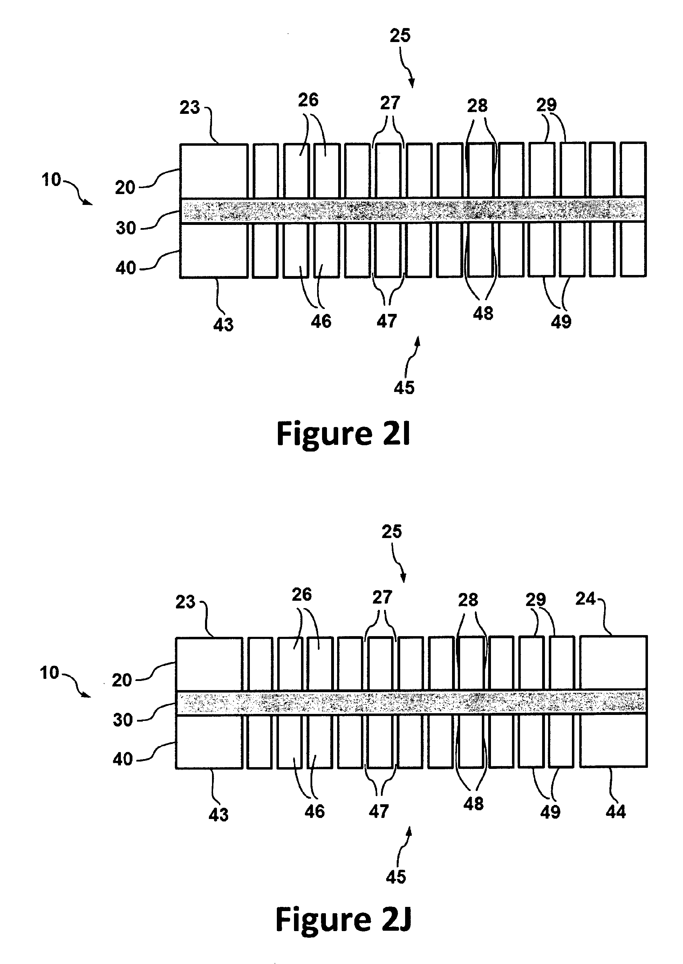

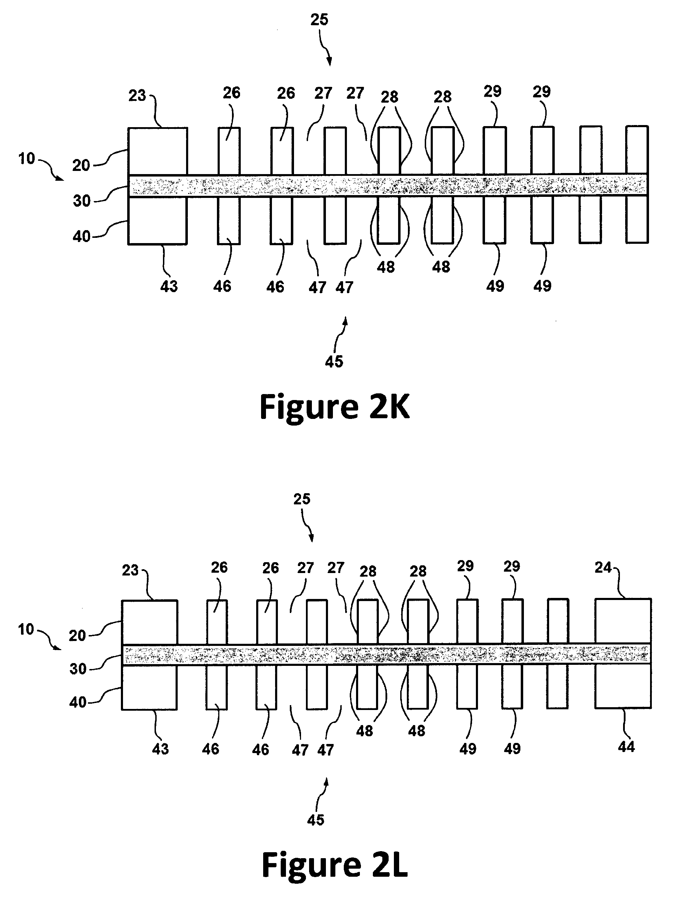

The elastic laminate (10) can also include a second fabric layer (40) as seen in Figures 2A - 2L. In this embodiment, the elastic layer (30) is the innermost part of the laminate (10) wherein the fabric layers (20, 40) are the outermost parts. The second fabric layer (40) has an elastic layer (30) contacting surface (41) and an elastic layer (30) non-contacting surface (42). The second fabric layer (40) may (Figures 2B, 2D and 2I-2L) or may not (Figures 2A, 2C and 2E-2H) be cut into several pieces (46) forming apertures (47) wherein the fabric pieces (46) have recessed (48) and protruding sides (49). The second fabric layer (40) should be made from an expandable material if it is not activated. If the second fabric layer (40) was cut, it would have at least one functional zone called first functional zone (43) and preferably second functional zone (44) and at least one activated zone (45). The elastic layer (30) may not be seen in the relaxed or retracted state of the laminate (10) (Figures 2A-2F, 2I and 2J) while it may be seen in the elongated state (Figures 2G, 2H, 2K and 2L).

The elastic layer (30) elongates upon an applied force and retracts when the force is removed in a direction (C) which is cross to the machine direction (M). The elastic layer (30) is made of an elastic material which can comprise elastomeric polymers such as block copolymers comprising SBS and/or SEBS, styrene block copolymers, polyesters, polyurethanes, polyethers and polyether block copolymers among others. Here "elasticity" or related terms describe the behavior of an elastic material upon application and removal of a force. Size of an elastic material can change dramatically when a force is applied in at least one direction. For example, an elastic material can elongate by at least 200 % of its original length, which is the length in a relaxed state when no force is applied, when a force is applied. After removal of applied force it can return to nearly its original length. For example, it can retract at least 50 % of its elongation.

The fabric layers (20, 40) have different characteristics from the elastic layer (30) in terms of elasticity. The fabric layers (20, 40) may be inelastic layers or in case of unapertured layers expandable layers which are single-layered and/or multi-layered web-like structures, more particularly nonwoven layers. These nonwoven layers can be made of polypropylene and/or its copolymers, polyethylene and/or its copolymers, cellulosic polymers and/or copolymers or mixtures of the said polymers, polyurethanes, polyesters, polyether or polyamide among others. The nonwoven materials can comprise spunbonded webs, bonded carded webs, airlaid webs, meltblown webs, hydroentangled webs, wet-formed webs, airthrough bonded webs or any combination thereof. The basis weight of the nonwoven layers can vary between 6 g/m² to 200 g/m². Rigidity of some nonwoven layers especially stiff bonded ones like spunbond and/or meltblown webs increase as the basis weight of the web increases. Rigidity of the fabric layer (20, 40) can be regarded as a resistance which hinders elongation of the stretchable laminate (10). Therefore, nonwoven layers having low basis weights (less than 50 g/m²) will be used as the fabric layer (20, 40) if the rigidity of the nonwoven layer increases upon the increase in its basis weight. Some types of nonwoven layers especially loose bonded ones like airthrough bonded and hydroentangled webs have a soft and bulky structure and rigidity of these nonwoven layers does not increase dramatically as the basis weight of the web increases even it can be an advantage forming more fluffy webs. Therefore, nonwoven layers having high basis weights (more than 50 g/m²) will be used as the fabric layer (20, 40) if rigidity of the nonwoven layer does not increase upon the increase in its basis weight.

The drawings are not scaled and shown in bigger sizes than the original dimensions in order to simplify the explanation. The thickness of the layers (20, 30, 40) can vary between about 5 micrometers to 4 millimeters. As mentioned above nonwoven layers especially loose bonded ones like airthrough bonded and hydroentangled webs have a bulky and fluffy structure which increases the width of the web. These types of webs can have a width of a few millimeters. Conversely, nonwoven layers especially stiff bonded ones like spunbond and/or meltblown webs consist of thin layers of a few microns which have a flat and smooth surface. The drawings make someone to have a perception that adjacent layers seem apart from each other but the innermost surface (21) of the first fabric layer (20) contacts to the first surface (31) of the elastic layer (30) and the innermost surface (41) of the second fabric layer (40) contacts to the second surface (32) of the elastic layer (30).

The apertures (27, 47) can be die-cut, kiss-cut, slit, scored, laser cut, water jet cut or otherwise sharply spaced. The recessed sides (28, 48) and thereby protruding sides (29, 49) of the fabric pieces (26, 46) of the fabric layers (20, 40) can be formed in this way. The length of the first fabric layer (20), the length of the second fabric layer (40) and the length of the elastic layer (30) can be identical along the machine direction (M). The length of the activated zone (25, 45) can be equal to the length of its fabric layer (20, 40) and the length of the elastic layer (30). The width of the first fabric layer (20) and the width of the second fabric layer (40) can be identical along the cross direction (C). The width of the elastic layer (30) can be identical to or different from the width of the fabric layer (20, 40) along the cross direction (C).

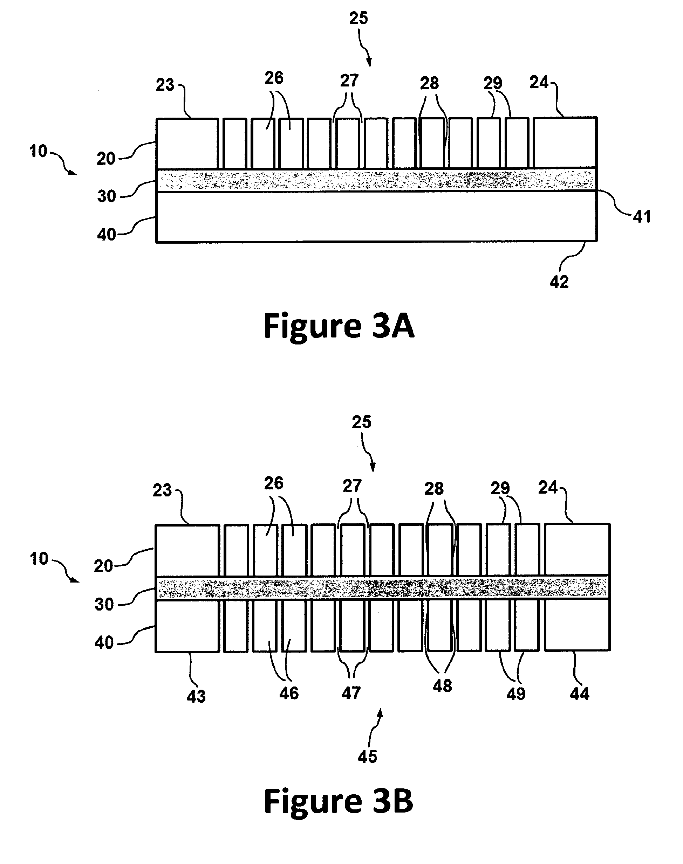

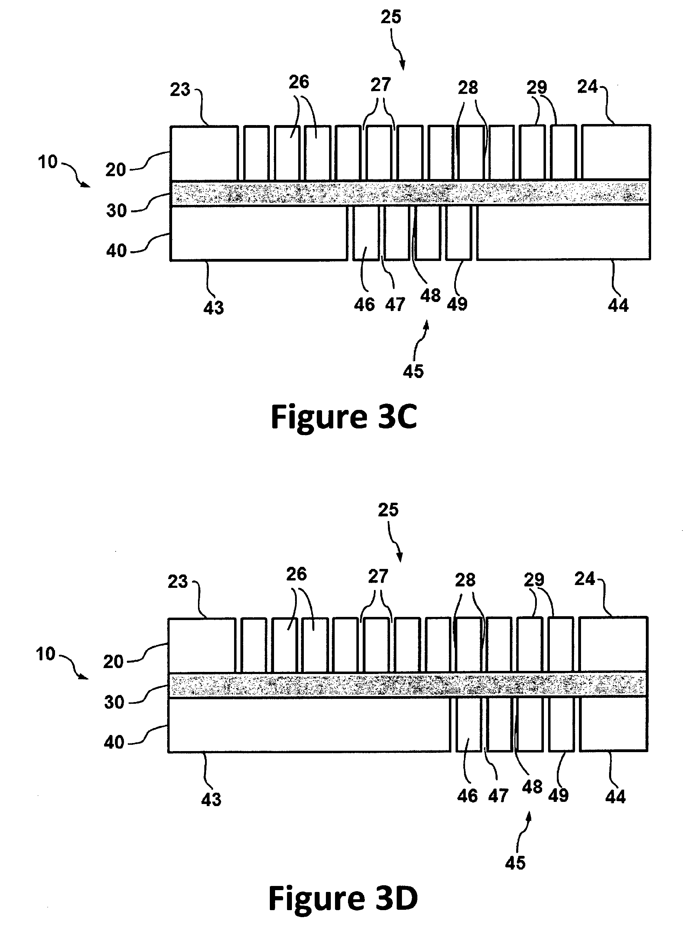

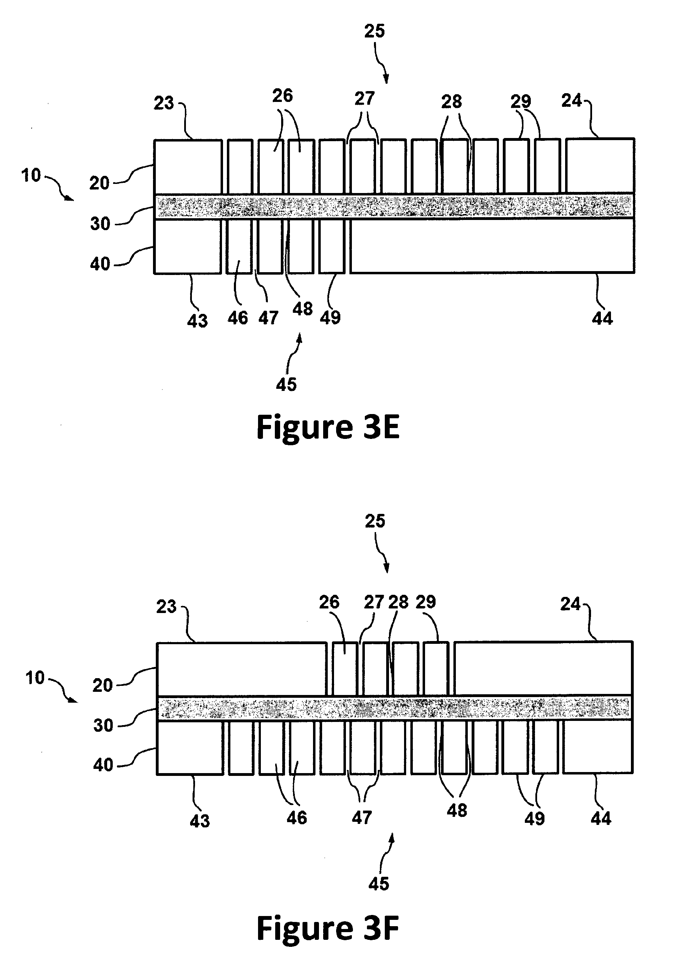

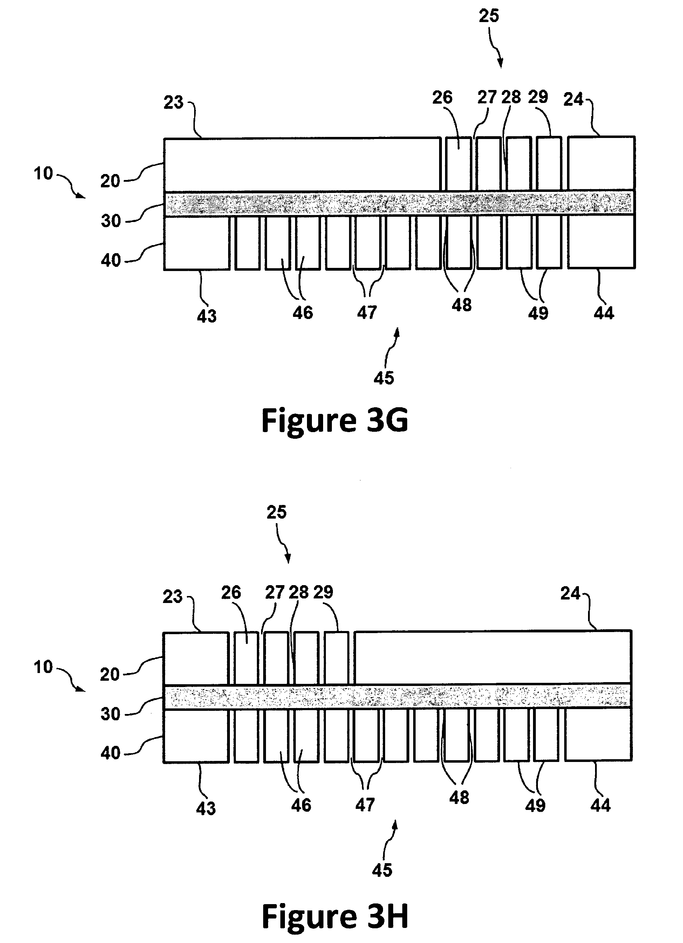

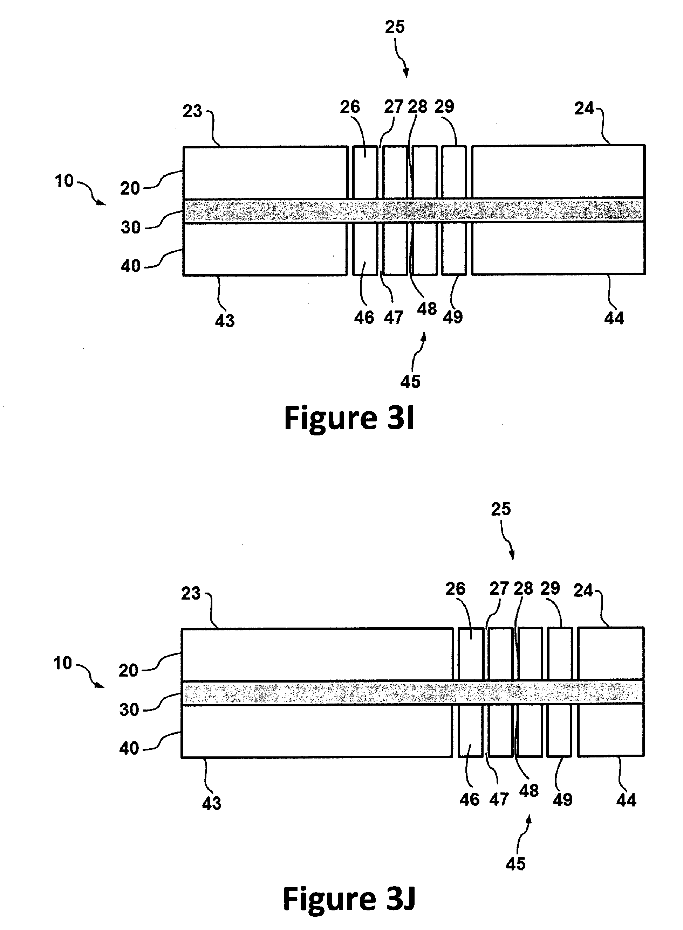

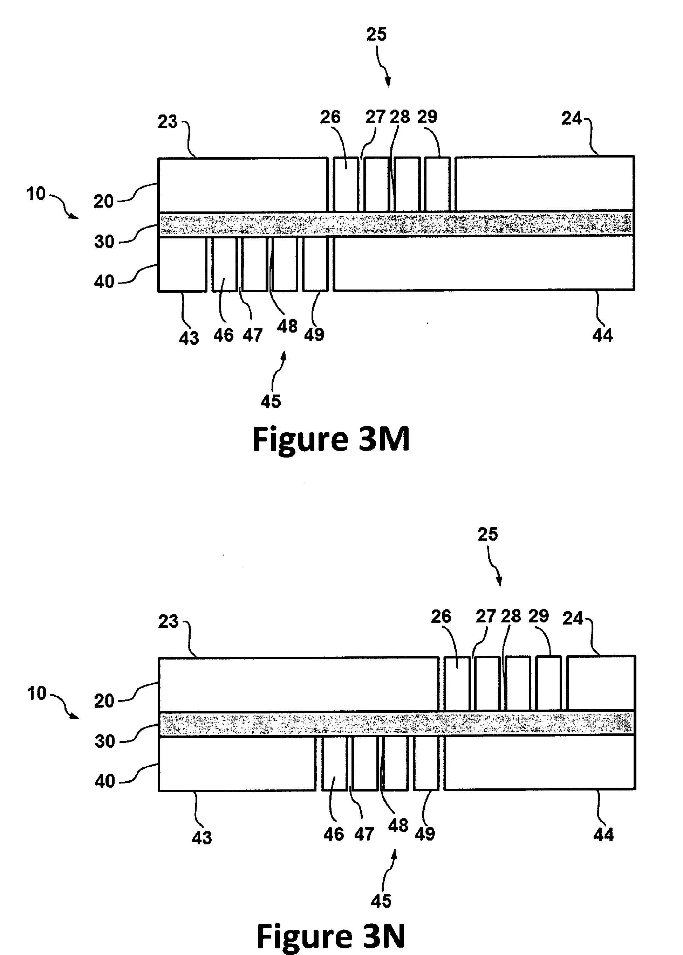

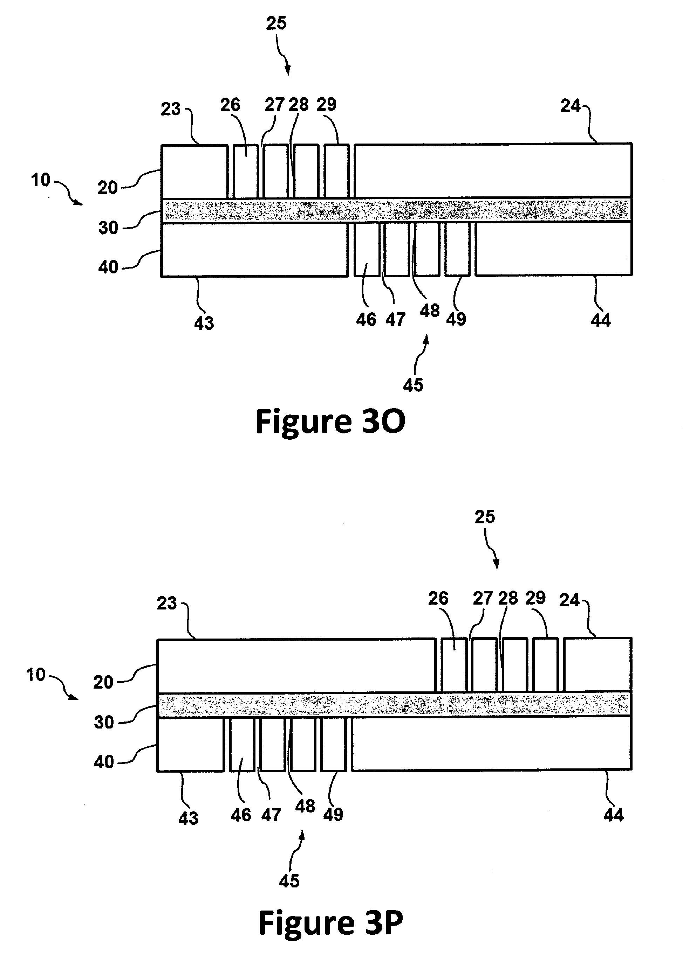

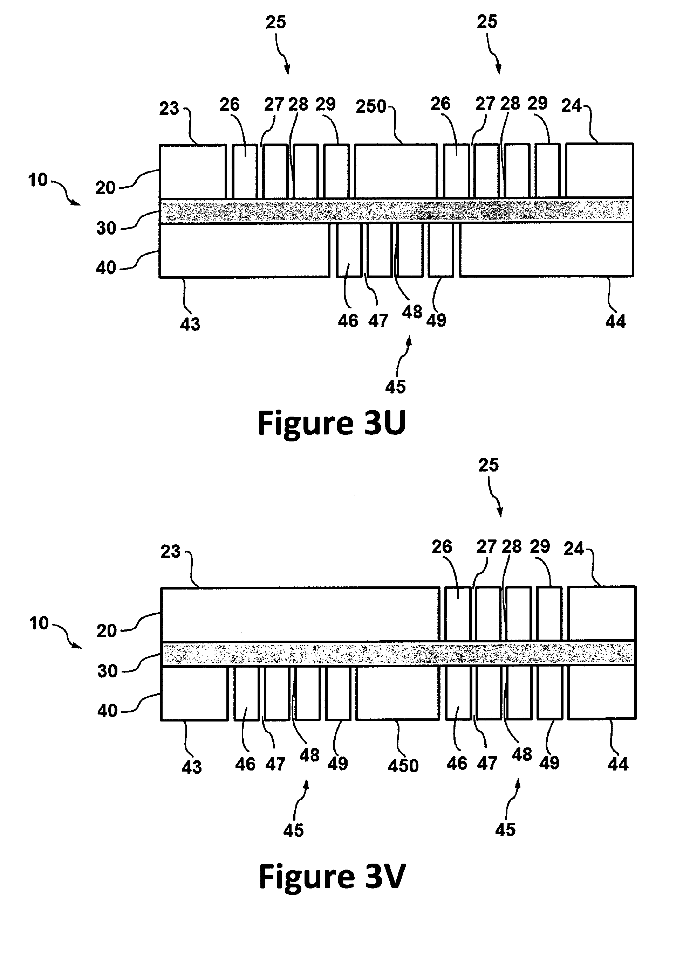

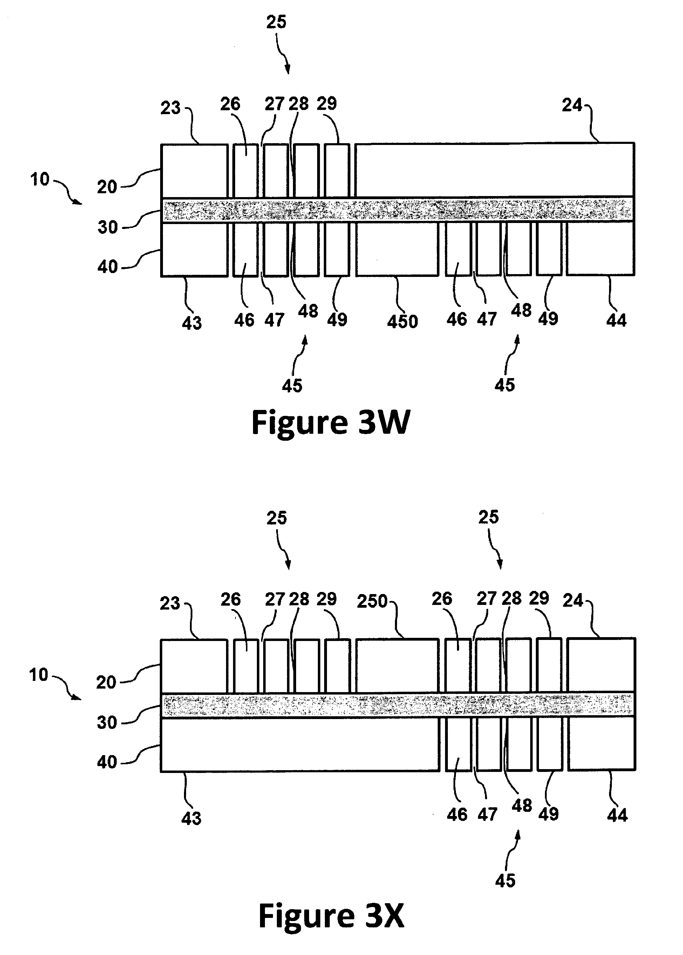

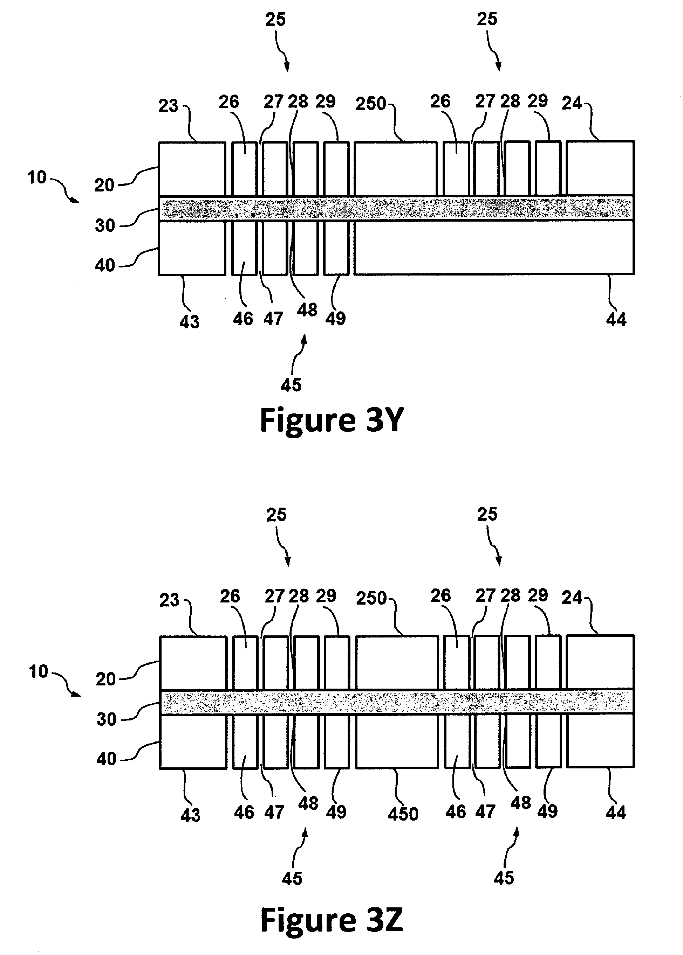

The width and the position of the activated zone (25, 45) in the cross direction (C) can be variable depending on the degree of elasticity required. Thus, some possible designs are shown in Figures 3A-3Z. If the second fabric layer (40) did not have an activated zone (45) the width of the activated zone (25) of the first fabric layer (20) would reach its maximum value (Figure 3A). If both of the fabric layers (20, 40) have activated zones (25, 45) the width of the activated zone (25) of the first fabric layer (20) can be equal to the maximum width which the activated zone (25) in Figure 3A can have (Figures 3B-3E) or smaller (Figures 3F-3Q). Similarly, the activated zone (45) of the second fabric layer (40) can have the same width with the activated zone (25) in Figure 3A (Figures 3B and 3F-3H) or may be smaller (Figures 3C-3E and Figures 3I-3Q). In the designs shown in Figures 3C-3H the narrower activated zone can be aligned to the middle or the right hand side or the left hand side of the broader activated zone or it can be randomly placed.

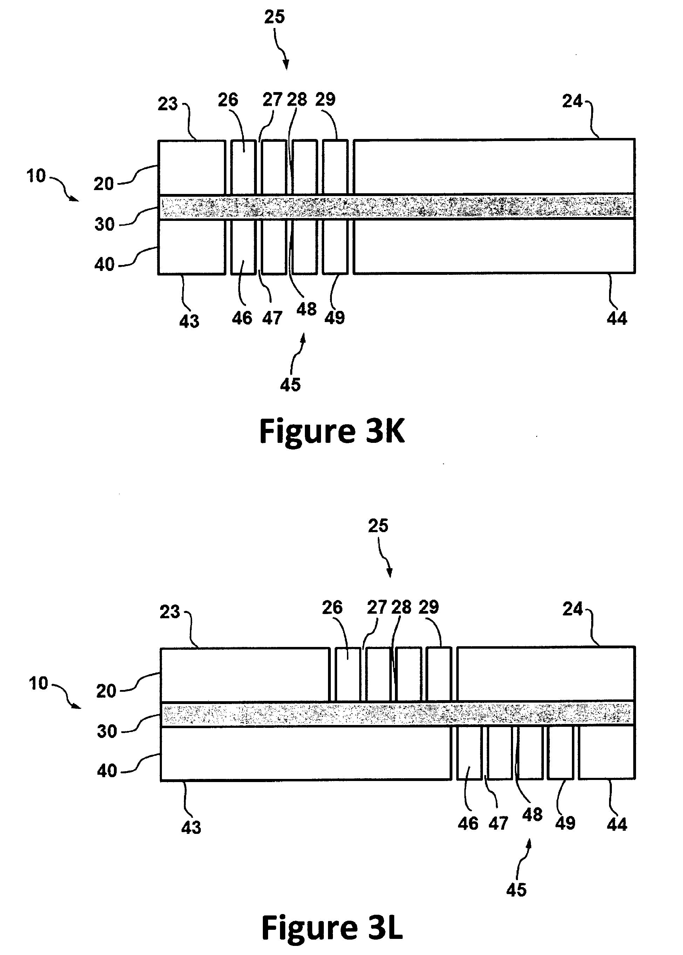

The widths of both of the activated zones (25, 45) can be smaller than the maximum width which the activated zone (25) in Figure 3A can have (Figures 3I-3Q), the width of the one of the activated zones (25, 45) can be equal to the width of the other activated zone (25, 45) or bigger or smaller. The activated zones (25, 45) can be aligned to each other in the middle part (Figure 3I) or on the right hand side (Figure 3J) or on the left hand side (Figure 3K) of the fabric layers (20, 40) or randomly arranged (Figures 3L-3Q). If there is difference between the widths of the activated zones (25, 45) the narrower activated zone can be aligned to the middle or the right hand side or the left hand side of the broader activated zone or they can be randomly arranged. The activated zones (25, 45) can be arranged in an order wherein they can intersect, overlap or be far away from each other.

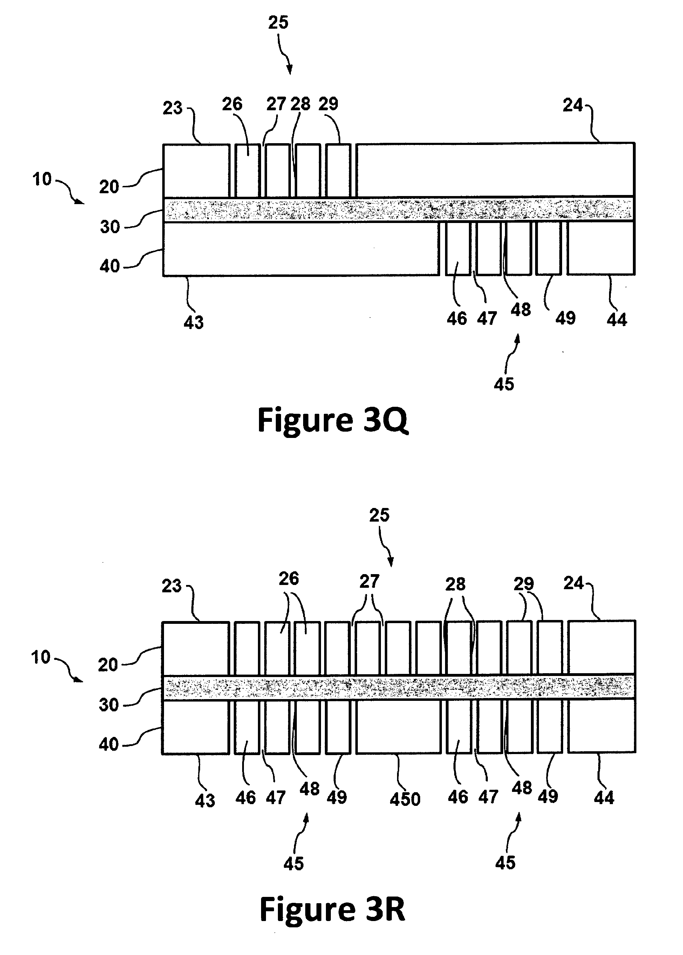

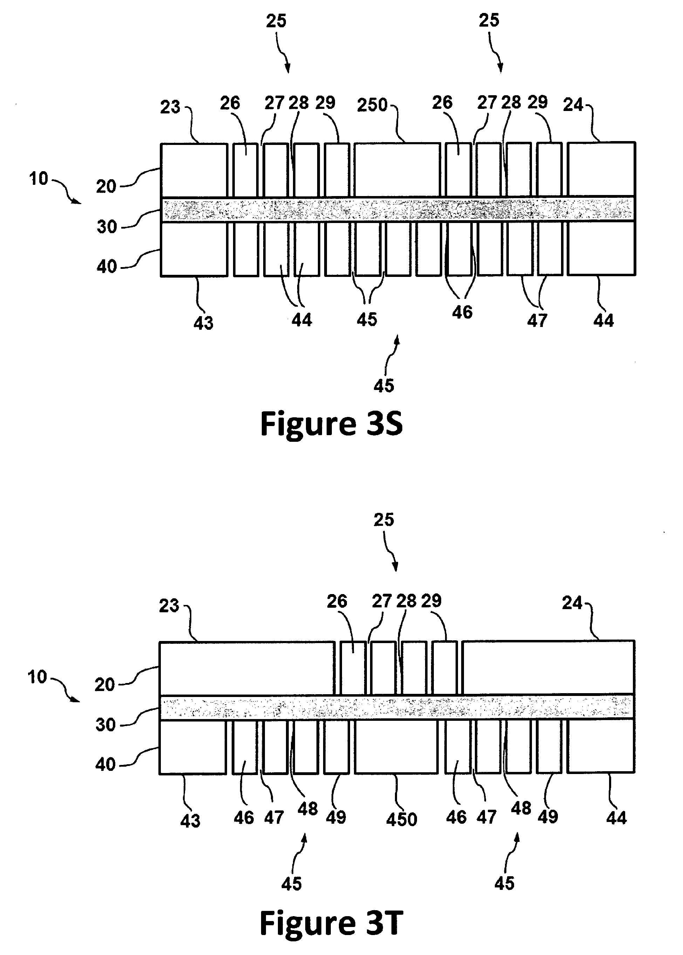

The fabric layers (20, 40) can have more than one activated zones (25, 45) (Figures 3R-3Z). At least one fabric layer (20, 40) can have at least two activated zones (25, 45) (Figures 3R-3Y) or both of the fabric layers (20, 40) can have at least two activated zones (25, 45) individually (Figure 3Z). The width of the single activated zone (25, 45) can be equal to the maximum width which the activated zone (25) in Figure 3A can have (Figures 3R and 3S) or smaller (Figures 3T-3Z). In the designs shown in Figures 3T-3Z the widths of the activated zones (25, 45) can be identical to or different from each other. The activated zones (25, 45) of the fabric layers (20, 40) can be arranged in an order wherein they can intersect, overlap or be aligned each other. The multiple activated zones can be arranged in an order wherein at least one additional functional zone (250, 450) called third functional zone (250, 450) can be formed between them. The designs shown in Figures 3P, 3Q and 3V-3Z can also be used if the stretchable laminate (10) is manufactured in a form of a master roll. The master roll is cut into smaller rolls preferably at the middle of the third functional zone (250, 450). This can be preferable and advantageous when the manufacturing capacity is required to be increased.

The total elongation of the laminate (10) increases as the total width of the activated zone (25, 45) increases. Increase in the total width of the functional zone (23, 24, 43, 44) restricts elongation forming indeformable areas which can be regarded as a resistance to elongation. Laminates having different designs (Figures 3A-3Z) can be arranged in the order below in terms of degree of elasticity:

Figure 3B > Figure 3R and 3S > Figure 3Z ≥ Figure 3C-3H > Figure 3T-3Y > Figure 3A > Figure 3J-3Q

If the activated zones (25, 45) of the first fabric layer (20) and second fabric layer (40) are aligned to each other (Figure 3I-3K) a synergistic effect will occur which increases the total elongation of the laminate (10). If the activated zone (25, 45) of one of the fabric layers (20, 40) is aligned to the functional zone (23, 24, 43, 44) of the other fabric layer (20, 40) (Figures 3L-3Q) the total elongation of the laminate (10) will be less than the state which the activated zones (25, 45) are aligned to each other. Therefore; these three parameters, the width and position of the activated zones (25, 45) and the width of the functional zone (23, 24, 43, 44) can be adjusted to control the degree of elasticity.

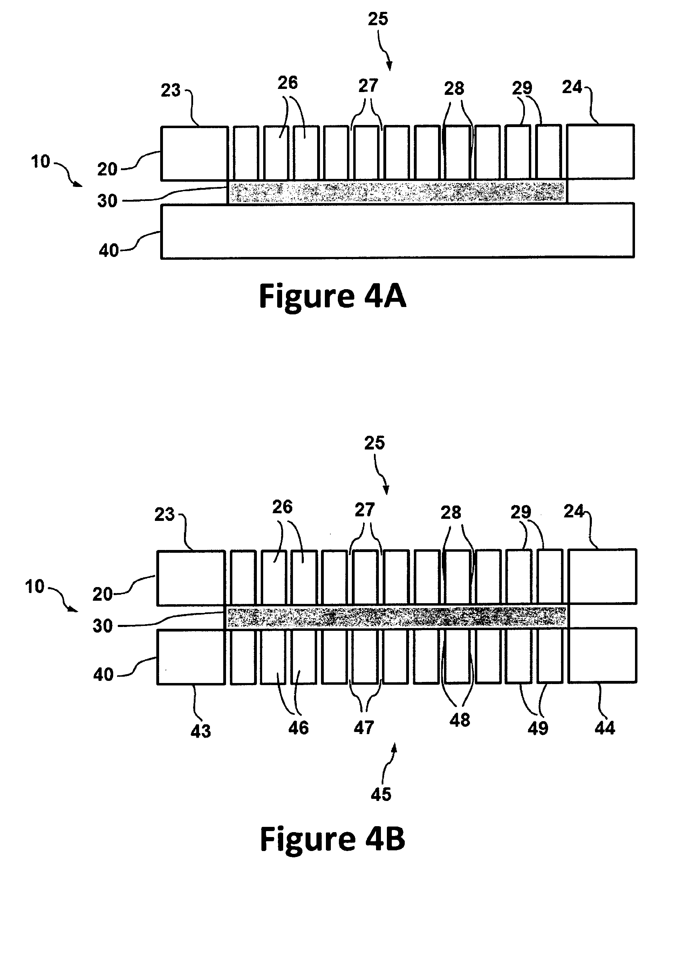

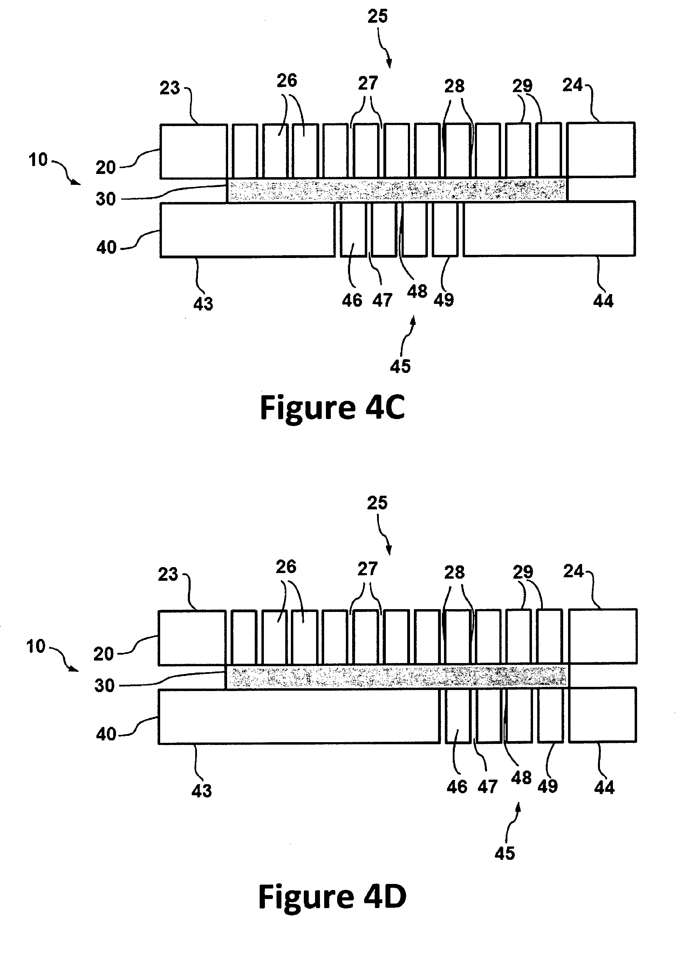

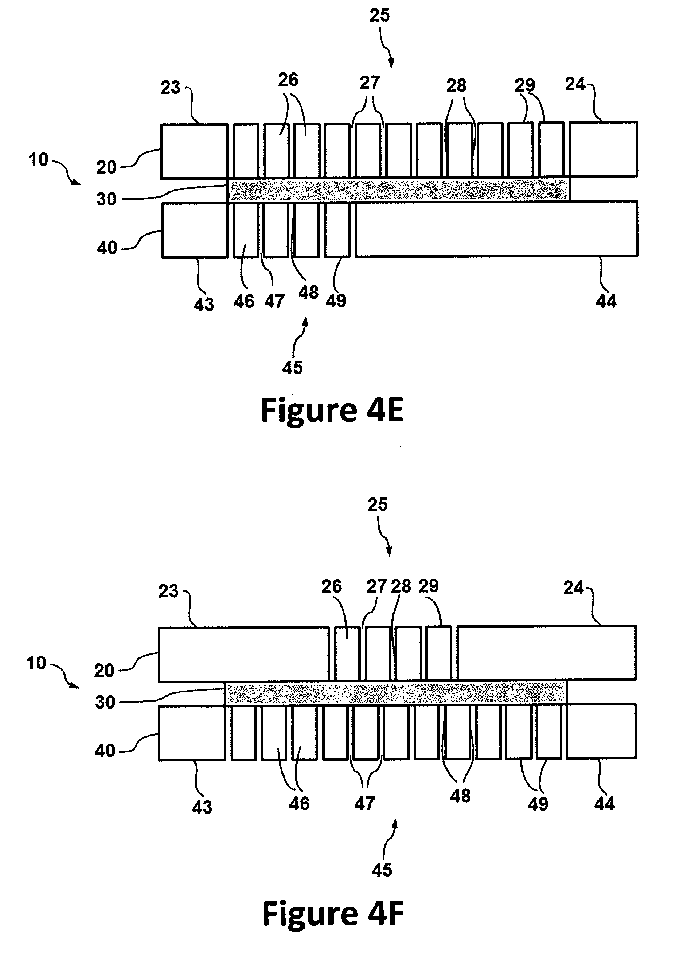

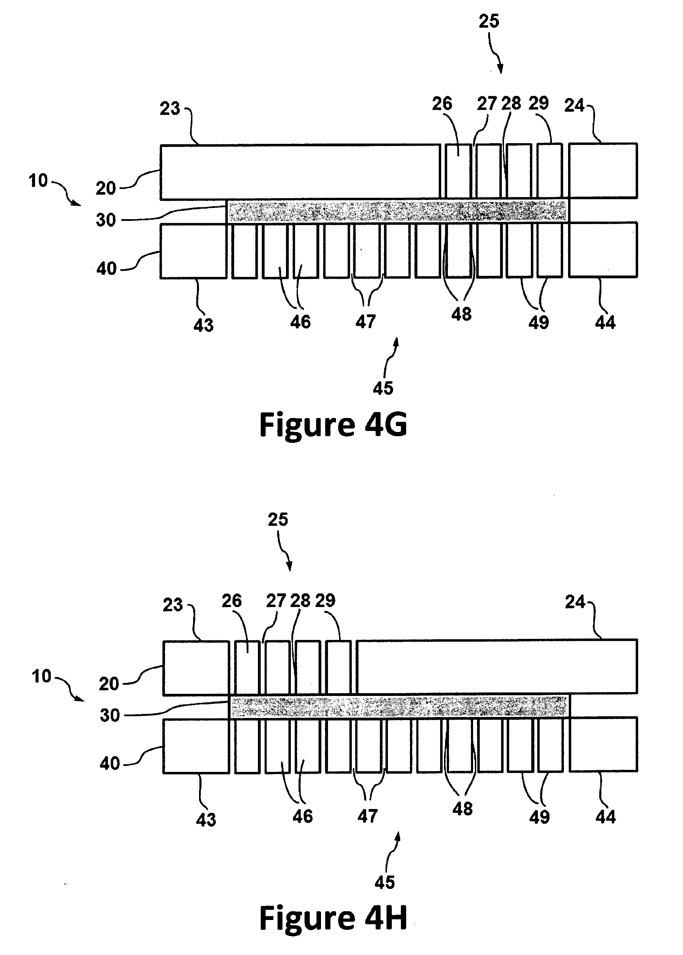

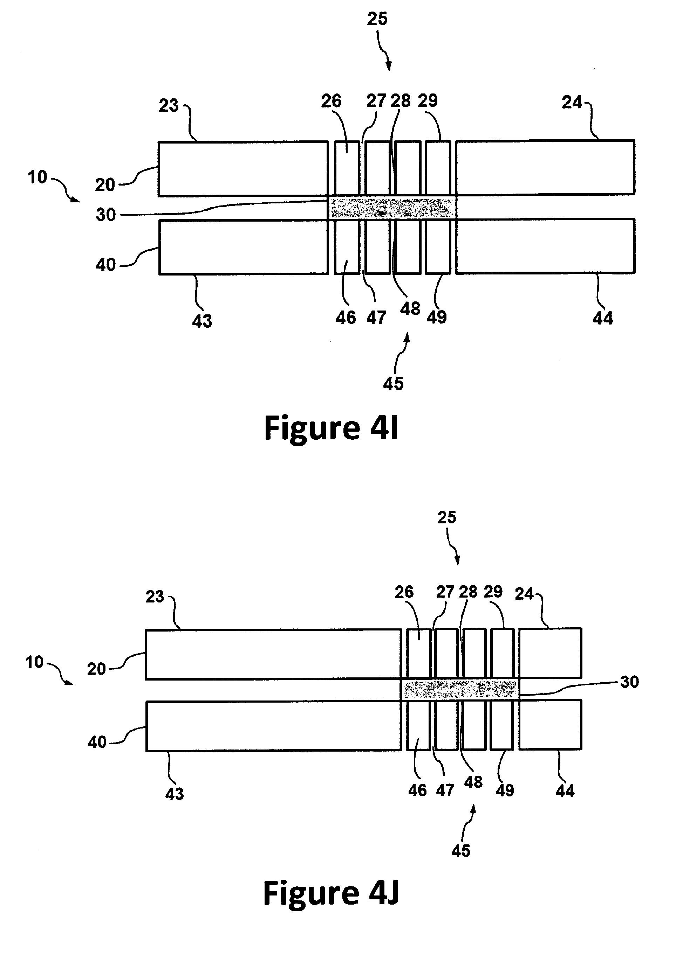

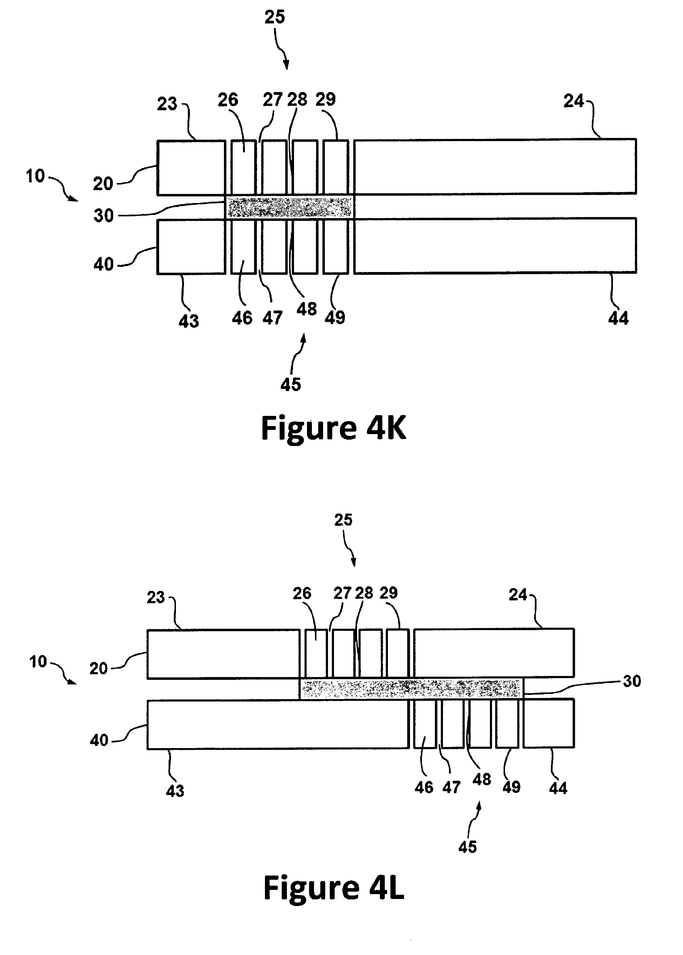

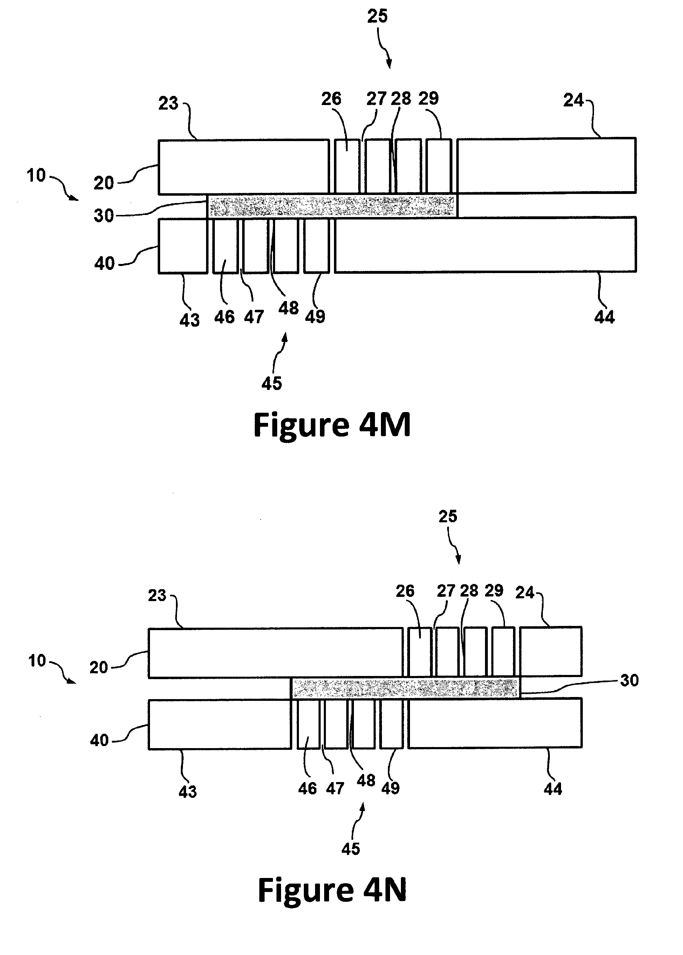

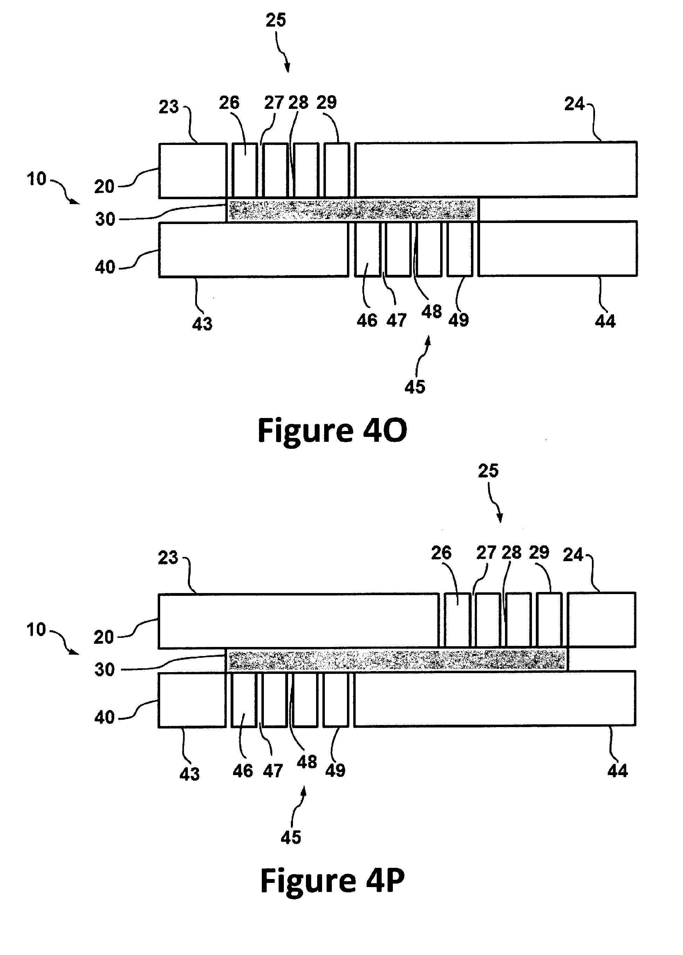

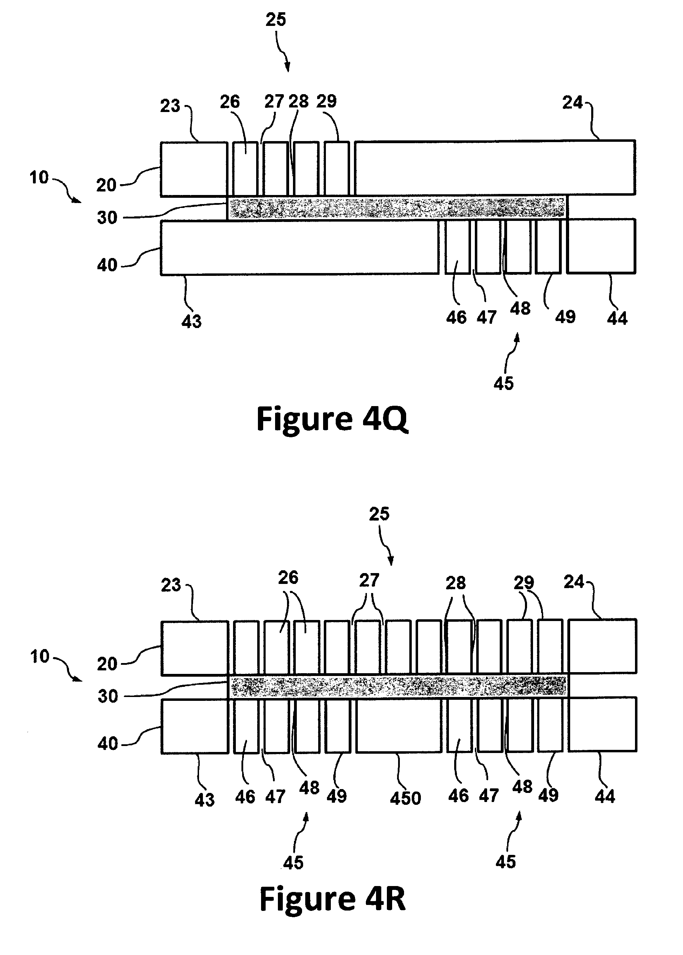

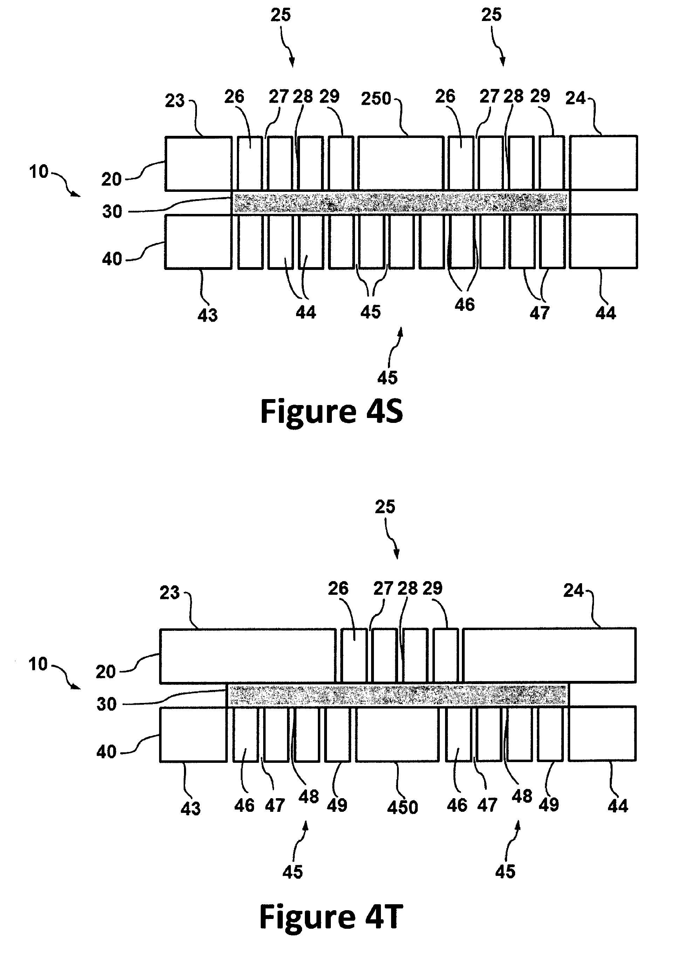

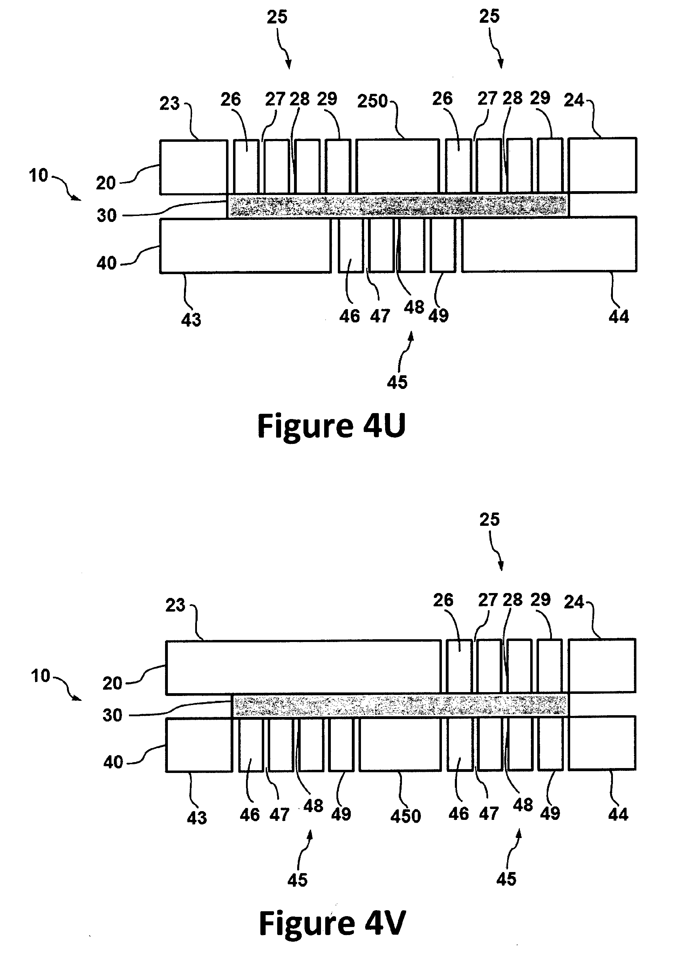

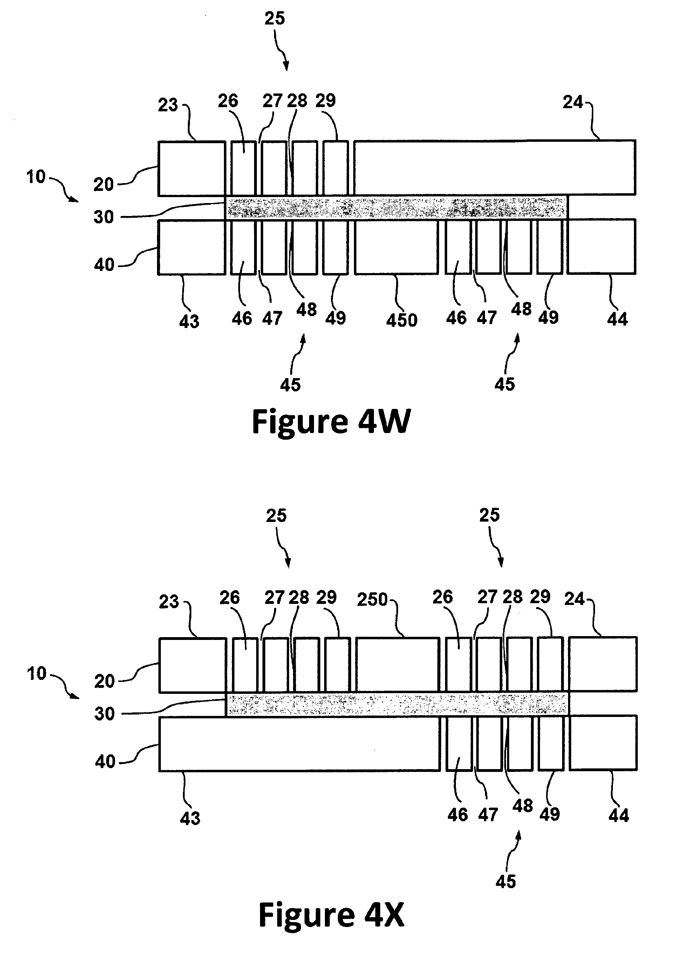

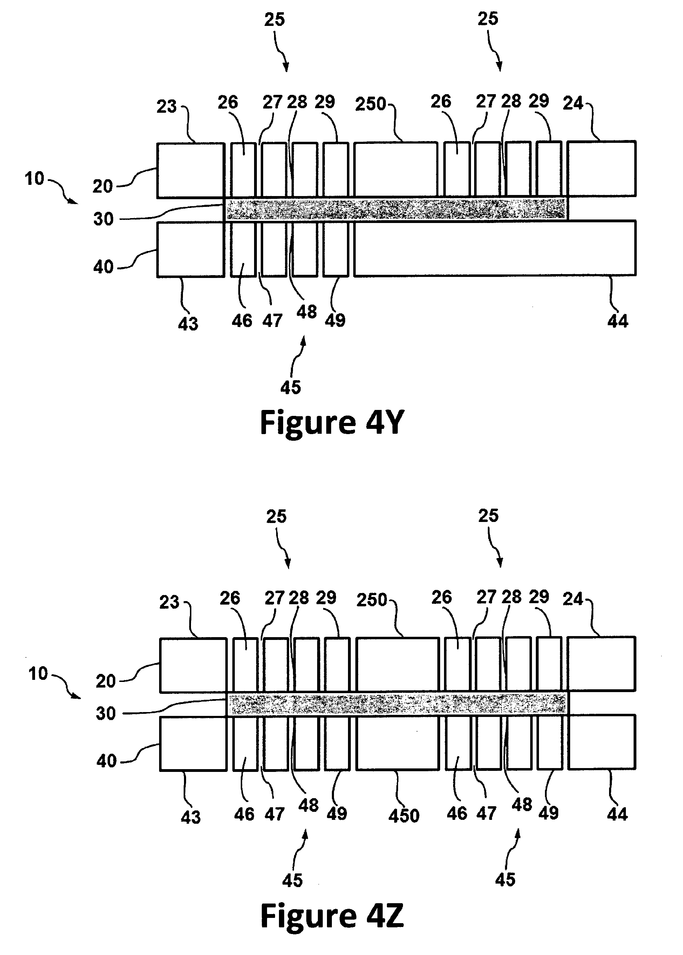

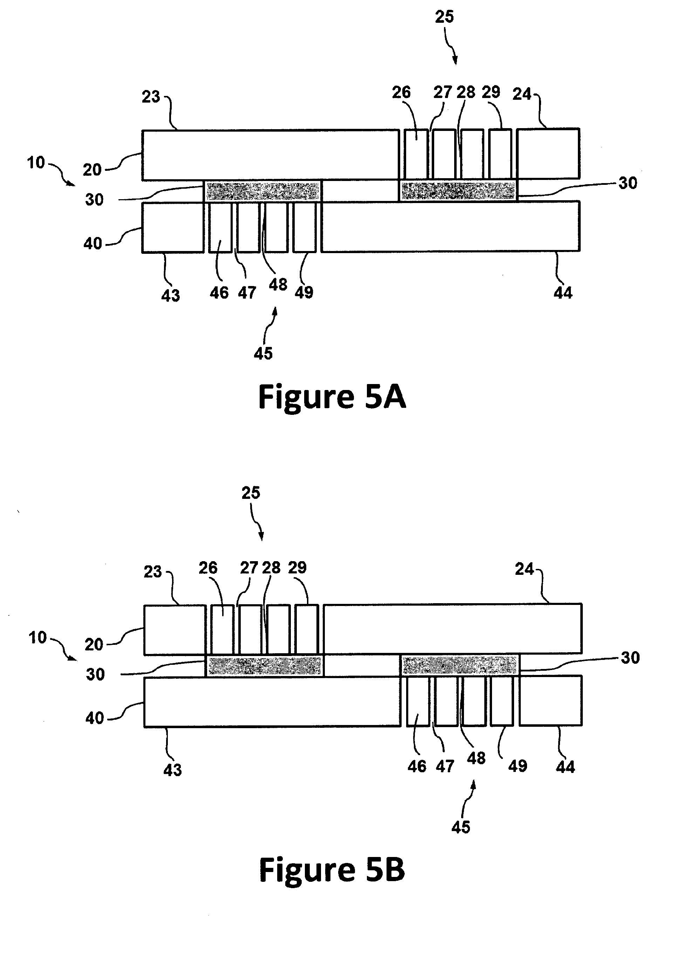

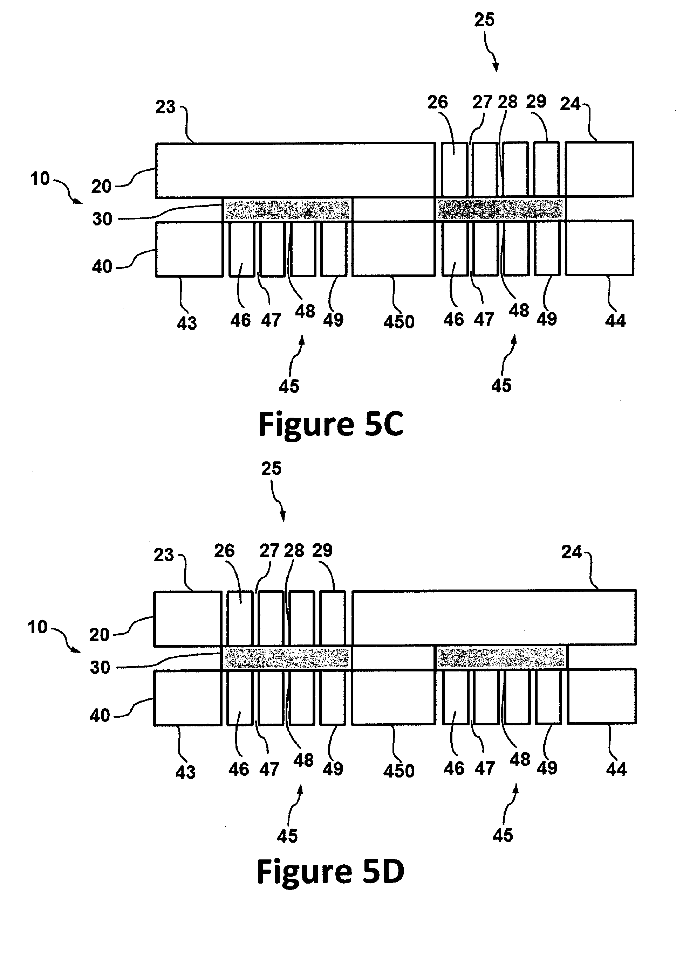

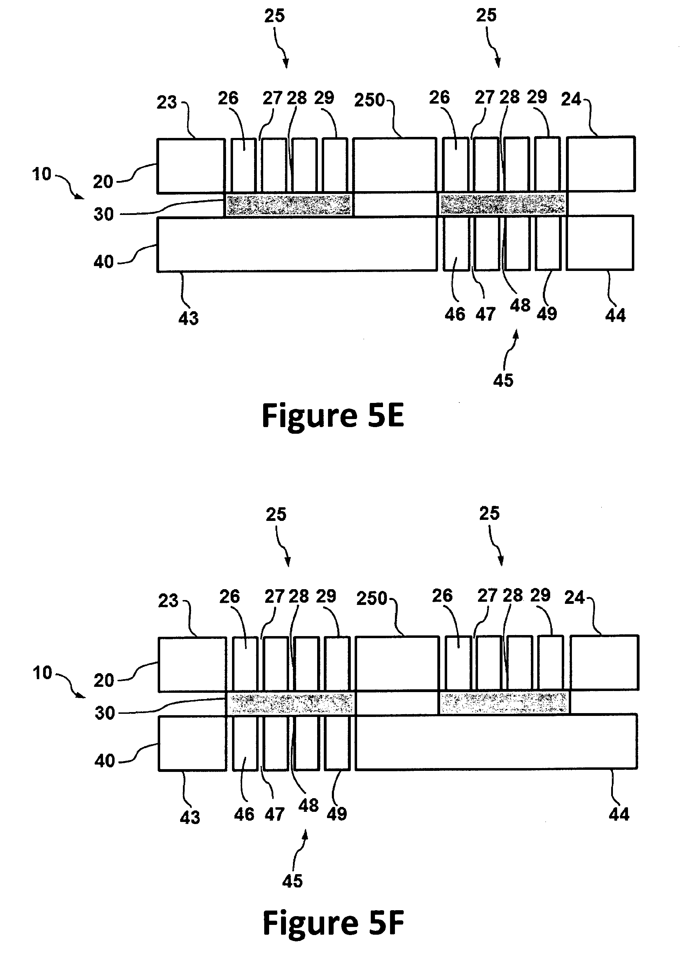

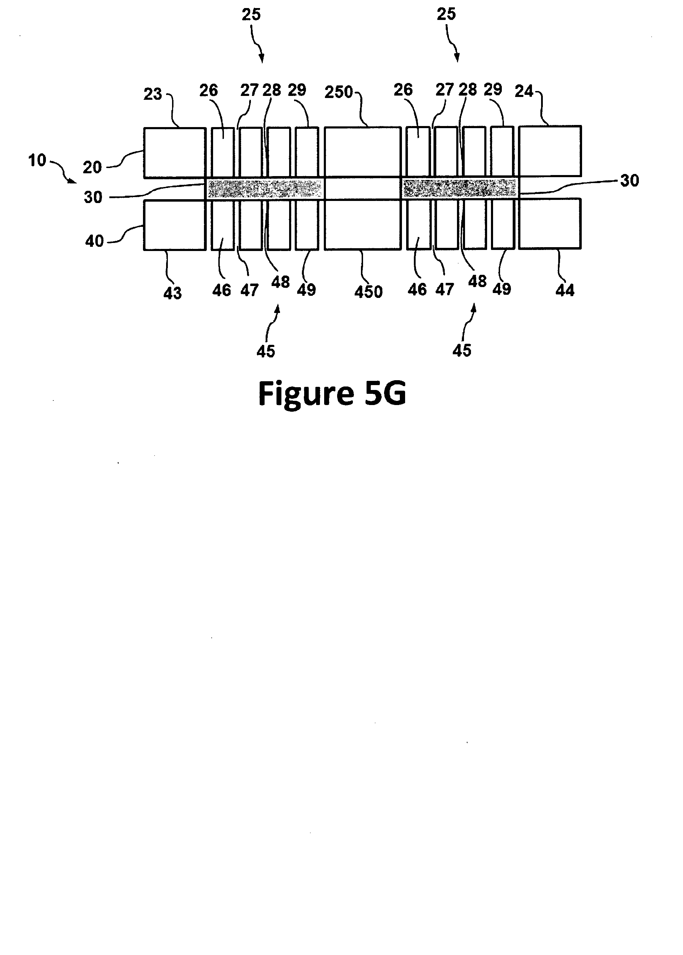

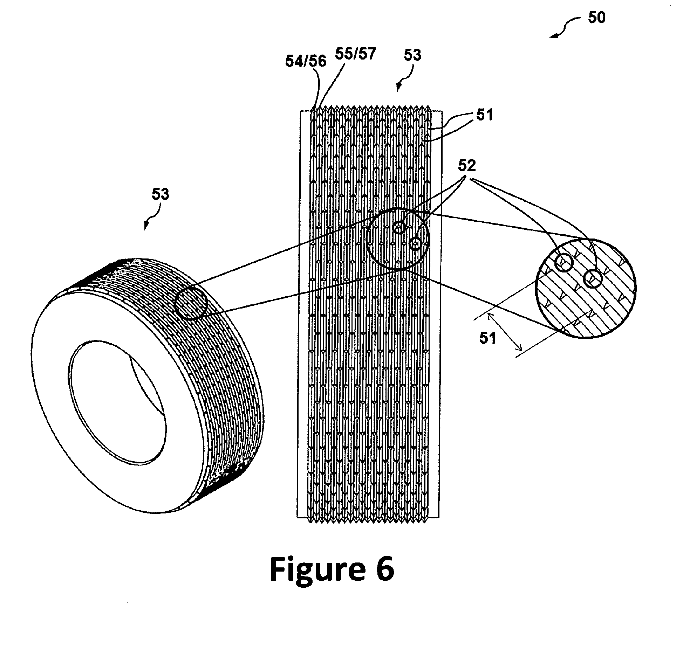

The possible designs wherein the width of the elastic layer (30) is smaller than the width of the fabric layer (20, 40) are shown in Figures 4 and 5. The elastic layer (30) can be continuous (Figures 4A-4Z) or intermittent (Figures 5A-5G) through the distance between the distal edges of the activated zones (25, 45). In manufacture of the laminate (10) the innermost surfaces of the side edges of the fabric layers (20, 40) overlapping the areas which lack elastic layer (30) (Figures 5 and 6) are not treated with application of an adhesive or another bonding method and left blank intentionally for further processing. In manufacture of the absorbent articles these areas can be regarded as application areas which serve for open areas for adjoining the other components of the absorbent articles to the laminate (10). Additionally, the areas in the middle of the laminate (10) (Figures 5A-5G) which lack elastic layer (30) are necessary if the laminate (10) is manufactured in a form of master roll. These areas are formed for cutting the master roll as also mentioned before for the Figures 3P, 3Q and 3V-3Z. The elastic layer (30) is not placed in these areas in order to have the laminate (10) whose side edges are free of elastic layer (30) such as the ones shown in Figures 4A-4Z.

The aperture forming embodiment (50) is shown in Figure 6. The apertures (27) are formed by a plurality of notches (51) wherein each of them is located between two cavities (52) placed on a rolling assembly (53). The notches (51) are arranged in the form of dashed lines (54, 55) along the machine direction (M) wherein the interruptions through the dashed lines are (54, 55) provided by the cavities (52). The notches (51) in the first line (54) are not aligned to the notches (51) in the second line (55). The rest of the couples of the lines follow the same order with the primary couple of the first line (54) and second line (55) along the cross direction (C). The cavities (52) are arranged in the form of virtual lines (56, 57) which overlap the dashed lines (54, 55) of the notches (51). The cavities (52) in the first virtual line (56) are not aligned to the cavities (52) in the second virtual line (57). The rest of the couples of the virtual lines follow the same order with the primary couple of the first virtual line (56) and second virtual line (57) along the cross direction (C).

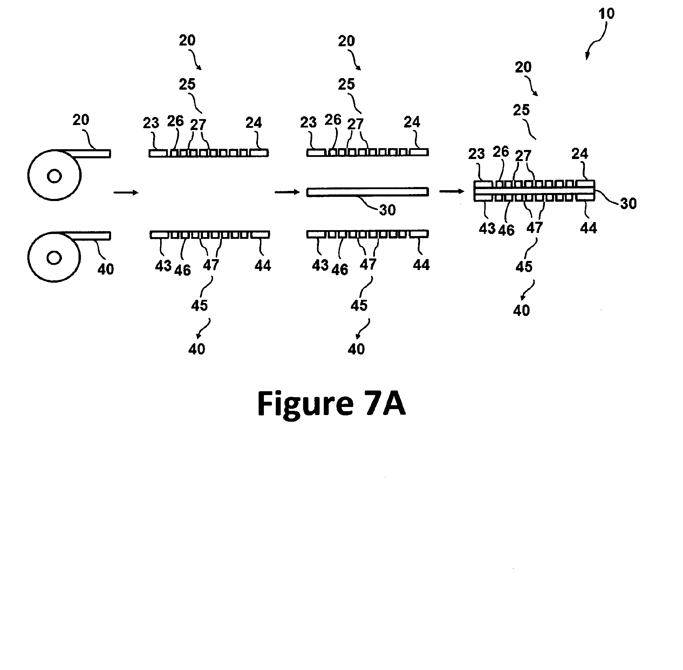

A schematic drawing of method of manufacturing the elastic laminate (10) is shown in Figure 7A. The method comprises forming the activated zone (25, 45) on the fabric layer (20, 40) by aperturing and laminating the fabric layer (20, 40) to the elastic layer (30). The tensile forces which will be applied to the layers (20, 30, 40) in the manufacturing will be in the range so as to be enough to make them run through machinery. The layers (20, 30, 40) will not be strained further for activation or any other purposes. The elastic layer (30) and fabric layer (20, 40) are endlessly fed and then apertures (27, 47) are formed on the fabric layer (20, 40) before lamination. The apertures (27, 47) can be formed by die-cut, kiss-cut, slit, scored, laser cut, water jet cut or otherwise sharply spaced. If the second fabric layer (40) of the laminate (10) did not have the activated zone (45) there would be no need to operate the activation step of the processing of the second fabric layer (40). If the elastic laminate (10) did not have the second fabric layer (40) there would be no need to operate the processing step of the second fabric layer (40) of the lamination process.

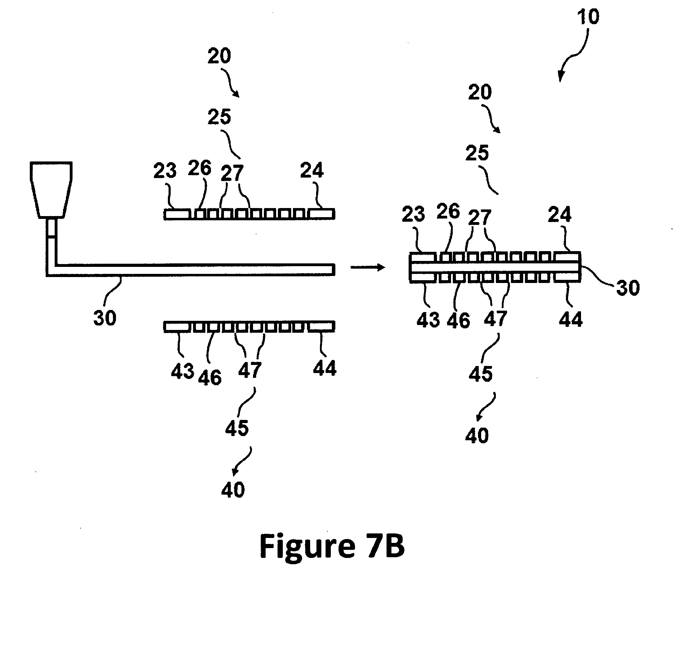

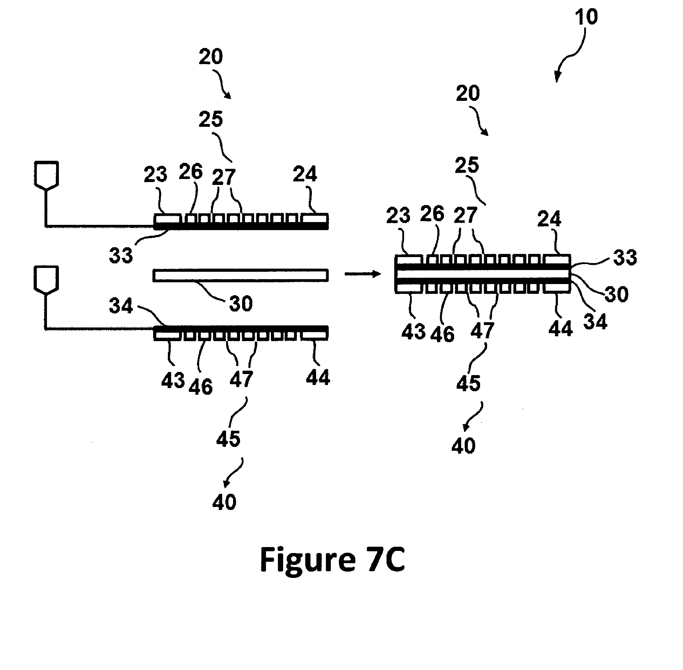

In lamination step, extrusion (Figure 7B) or non-extrusion techniques (Figures 7C-7E) can be used. In extrusion lamination (Figure 7B), thermal and physical properties of the polymeric melt are optimized to have both good bonding stability and stretchability of the elastic layer (30).

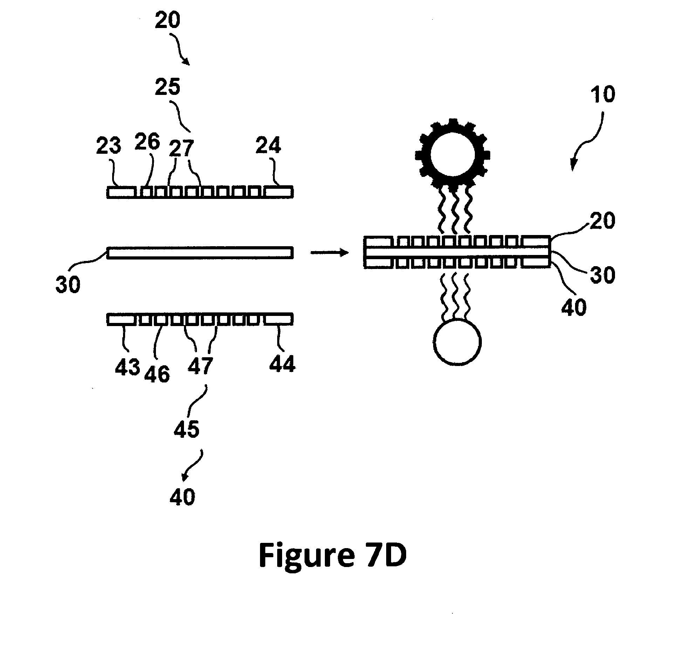

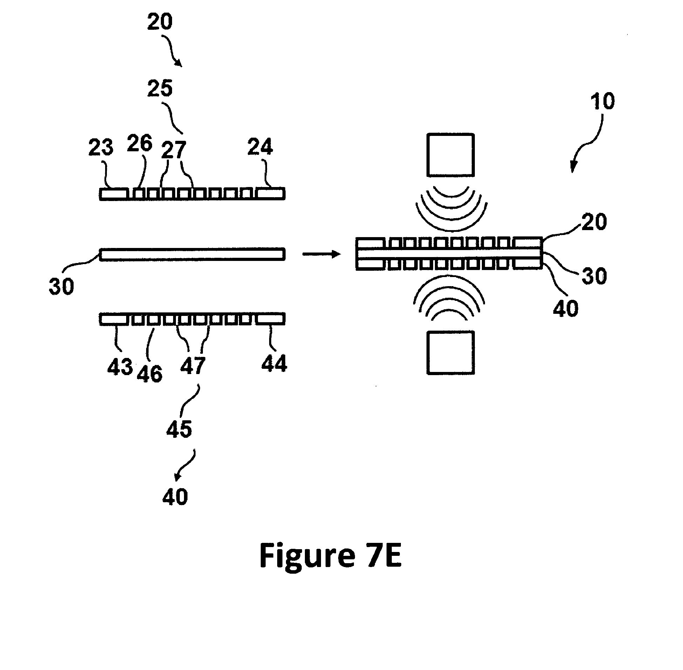

One of the non-extrusion techniques for lamination is adhesive lamination. In adhesive lamination (Figure 7C), an adhesive sub-layer (33, 34) is formed on the fabric layer (20, 40) wherein the adhesive sub-layer (33, 34) is sandwiched between the fabric layer (20, 40) and the elastic layer (30). The adhesive can comprise hot-melt adhesives such as rubber-based materials or polyolefin derivatives and /or non-hotmelt adhesives such as pressure sensitive adhesives. The adhesive can be applied to the surface of the fabric layer (20, 40) in a continuous pattern covering the whole bonding surface of the fabric layer (20, 40) or discontinuous patterns such as stripes, spots, swirls, grids or random via contacting (e.g., slot, kiss roll) or non-contacting (e.g., spraying) methods. Other non-extrusion techniques can be mechanical bonding techniques such as thermal (Figure 7D) or ultrasonic bonding (Figure 7E).

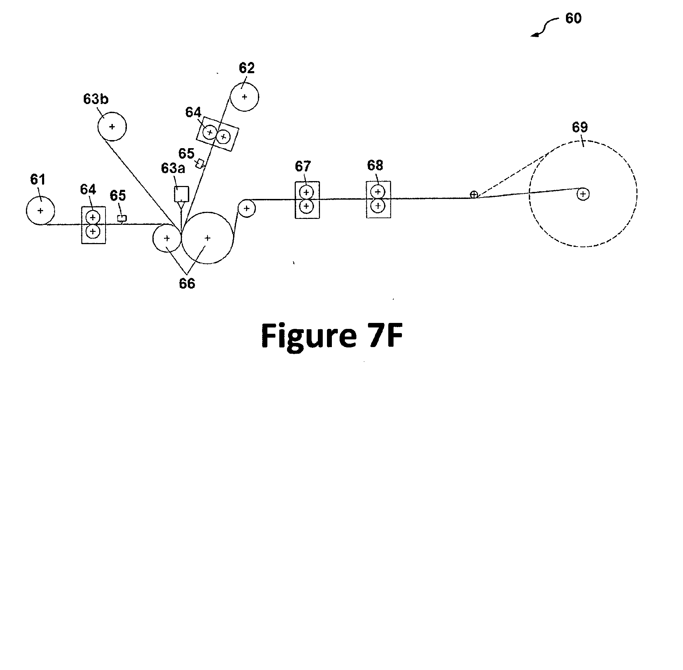

An example of illustration of the manufacturing process (60) of the elastic laminate (10) is shown in Figure 7F. The fabric layers (20, 40) are fed to the machinery through unwinding rollers (61, 62). If the elastic laminate (10) is manufactured by the extrusion lamination (Figure 7B), the elastic layer (30) is formed by an extrusion die (63a). If the elastic laminate (10) is manufactured by the adhesive lamination (Figure 7C) a roll of the elastic layer (30) is fed through another unwinding roller (63b). The apertures (27, 47) are formed on the fabric layer (20, 40) while it runs through the first activation unit (64). The first activation unit (64) consists of two rollers: the rolling assembly (53) having notches (51) and an anvil roller. The rolling assembly (53) having notches (51) is rotated on an anvil roller to form a plurality of apertures (27, 47) on the fabric layer (20, 40). If adhesive lamination (Figure 7C) is used the adhesive sub-layer (33, 34) is formed on the fabric layer (20, 40) by adhesive applicators (65). The layers (20, 30, 40) are brought together to form the laminate (10) while the pile of the layers (20, 30, 40) run through laminating calenders (66). The laminate (10) is optionally or further processed in mechanical bonding unit (67). In this unit thermal (Figure 7D) or ultrasonic bonding (Figure 7E) can be used for lamination. The laminate (10) can be manufactured using only mechanical bonding without operating the adhesive applicators (65). The adhesive applicators (65) and mechanical bonding unit (67) can be co-operated using less adhesive amount compared to individual operation of the adhesive applicators (65). After lamination step the elastic laminate (10) is pre-conditioned by the second activation unit (68). The second activation unit (68) is different from the first one and used for the activation of the laminate (10) while the first one is used for the activation of the fabric layer (20, 40). The activation technique is also different from the first one and refers to pre-tensioning/pre-conditioning of the laminate (10) before use in order to prevent the wearer/mother/caregiver from having the perception of tearing of the laminate (10) in the first use. The endless layer of the elastic laminate (10) is rewound to have a roll of the elastic laminate (10) and/or slit into smaller rolls in slitting/ rewinding unit (69) if the laminate (10) is manufactured in a form of a master roll.

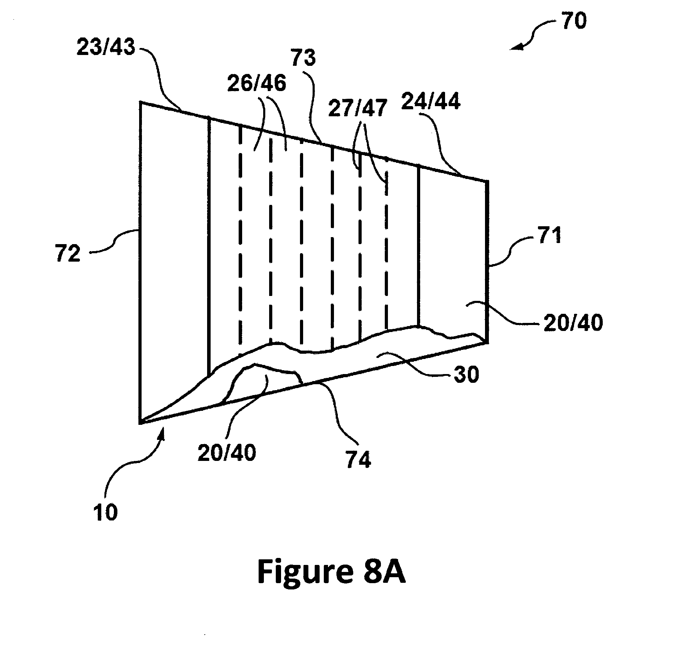

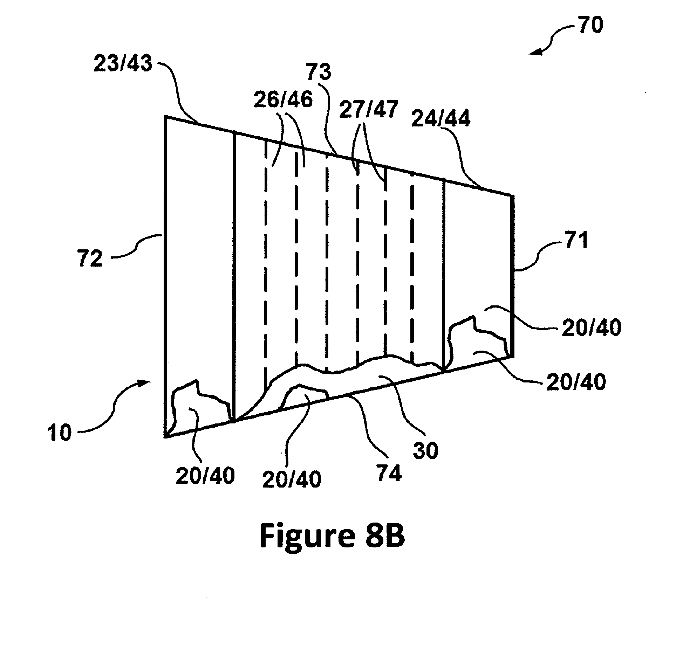

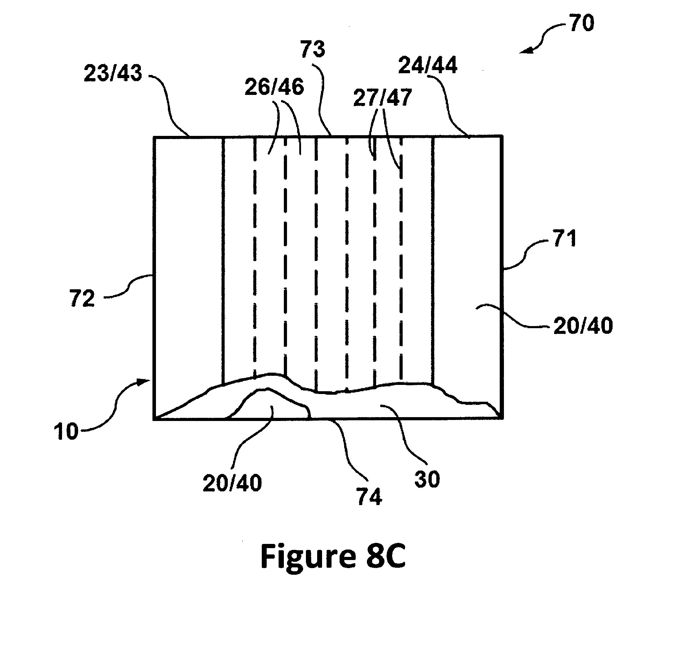

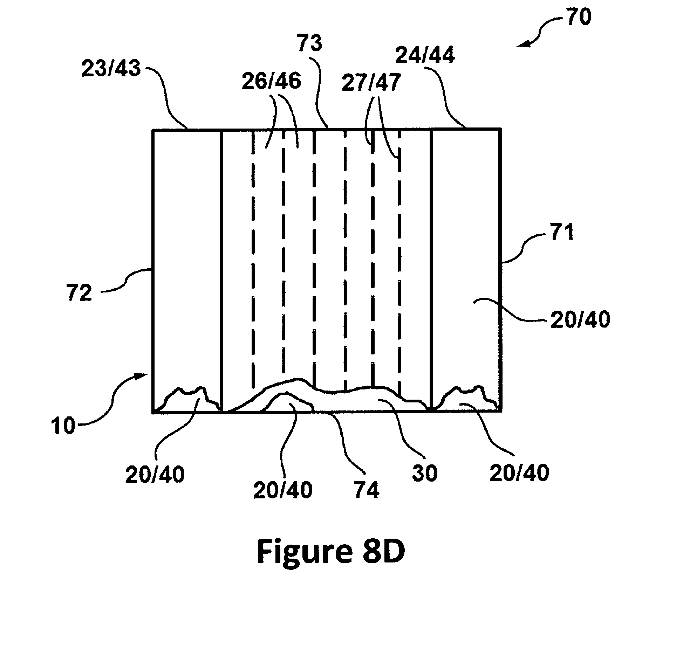

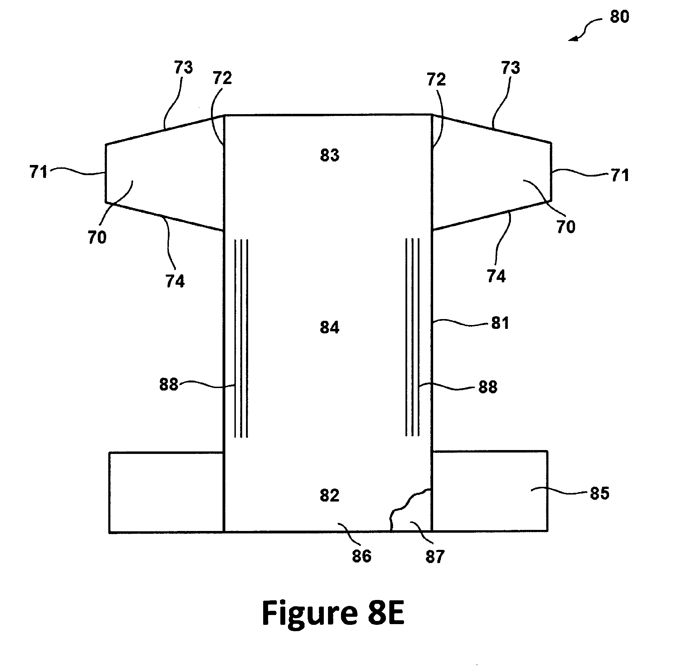

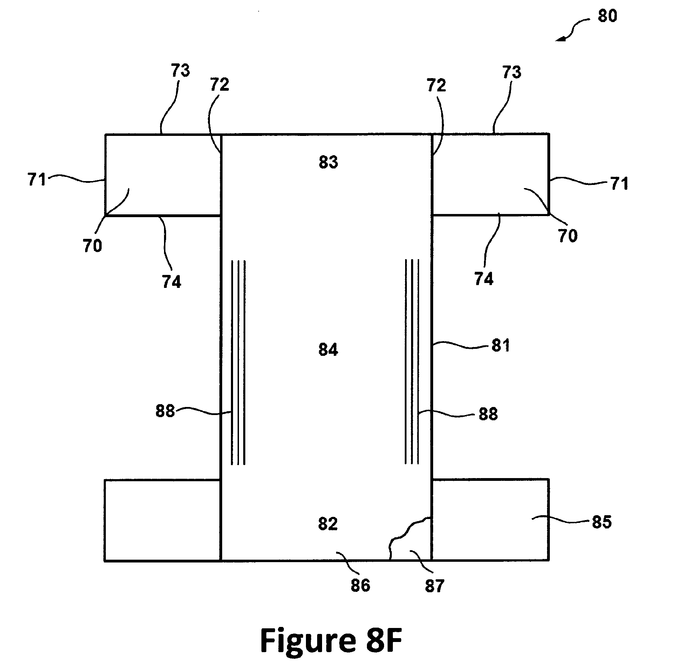

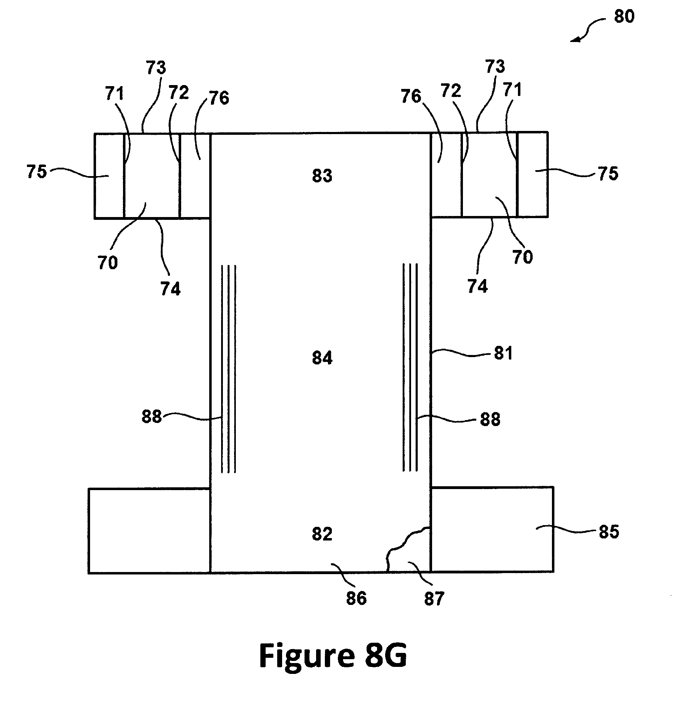

A rear side panel (70) including the elastic laminate (10) is shown in Figures 8A-8D. The side panel (70) comprises a terminal edge (71), a neighboring edge (72), an upper edge (73) and a lower edge (74). The side panel (70) can be a member of an absorbent article (80) (Figures 8E-8G) which comprises a chassis (81) including a front region (82), back region (83), a crotch region (84) between the front (82) and back region (83), front side panels (85), a garment facing layer (86), a body facing side (87) and leg elastics (88) between the garment facing layer (86) and body facing side (87). The lateral edges of the back region (83) are connected to the neighboring edges (72) of the side panel (70). The neighboring edge (72) of the side panel (70) can directly (Figures 8E and 8F) or indirectly (Figure 8G) connect to the lateral edge of the back region (83). The elastic laminate (10) can be cut in a shape of a trapezoid (Figure 8E) and used in this way as the rear side panel (70). It can also be used in a rectangular shape (in its original shape) individually (Figure 8F) or as an intermediate layer (Figure 8G) between two neighboring lateral inelastic fabric layers (75, 76) wherein one of the layers (76) are connected to the lateral edges of the back region (83) while the other one is connected to the terminal edge (71) of the side panel (70). The first fabric layer (20) or the second fabric layer (40) can be the skin facing side of the side panel (70) or the first fabric layer (20) or the second fabric layer (40) can be the outermost surface of the side panel (70). The distance between the two edges of the laminate (10) in the cross direction (C) coincide with the distance between the terminal edge (71) and neighboring edge (72) of the side panel (70). The machine direction (M) of the laminate (10) coincides with the distance between the upper (73) and lower edge (74). The side panel (70) is extensible in the direction of neighboring-terminal edge (72-71). One of the functional zones (23, 24, 43, 44) of the laminate (10) coincides with the terminal edge (71) and the other one coincides with the neighboring edge (72). The one which coincides with the neighboring edge (72) contributes to obtain adequate bonding ability and tear strength for the junction areas in which the neighboring edges (72) are connected to the lateral edges of the back region (83). The other functional zone (23, 24, 43, 44) which coincides with the terminal edge (71) serves for an application area for further processing such as attaching an additional component of the article (80) to the rear side panel (70). Similarly, this functional zone (23, 24, 43, 44) contributes to obtain adequate bonding ability and tear strength for the junction areas in which additional components are connected to the terminal edges (71). Additionally, rigidity of the functional zone (23, 24, 43, 44) in this area restricts application of force and prevents over-elongation. So, elongation and retraction of rear side panels (70) can be maintained at a desired level hindering deformation and favoring preservation of their shape.

The elastic laminate (10) can also be used as the garment facing layer (86). The elastic laminate (10) having only one fabric layer (20) wherein the fabric layer (20) is the garment facing surface of the garment facing layer (86) and the elastic layer (30) is the body facing surface of the garment facing layer (86) can be preferable as the body facing surface of the garment facing layer (86) does not touch skin of wearer. The functional zones (23, 24, 43, 44) coincide with the right and left hand side edges of the garment facing layer (86). The functional zones (23, 24, 43, 44) especially should not be activated when the elastic laminate (10) is used as the garment facing layer (86) due to existence of leg elastics (88) in these areas. Corrugations are formed in these areas because leg elastics (88) are always in strained position before use. So, activation of these areas would cause an unaesthetic appearance giving a perception that the material is accidentally torn in production and the product has a bad quality. Additionally, rigidity of these areas is required to obtain adequate bonding ability and tear strength as the front side panels (85) and rear side panels (70) are connected to the lateral edges of the front region (82) and back region (83), respectively.

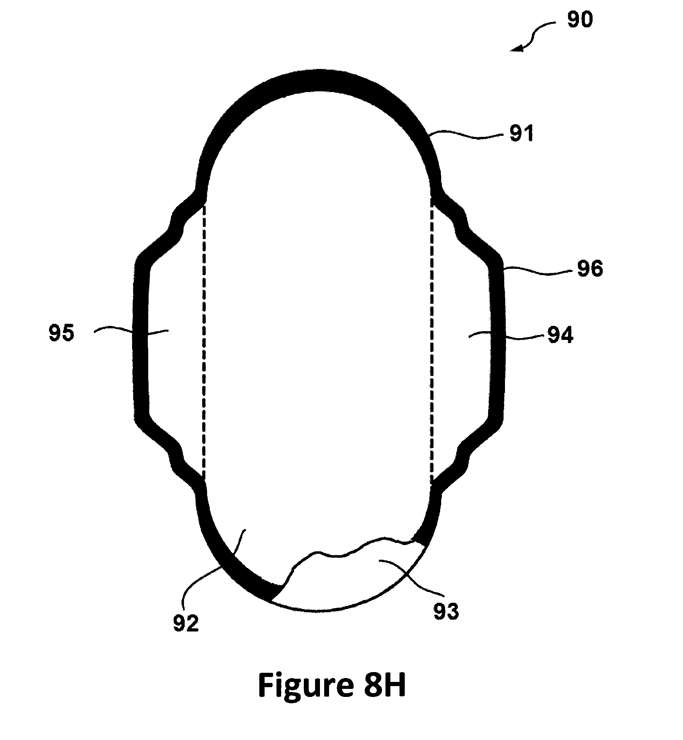

The elastic laminate (10) can also be a member of an absorbent article (90) (Figure 8H) which comprises a main body (91), a body facing layer (92), a garment facing layer (93), laterally extending side flaps (94, 95) and a sealed area (96) which encircles the contour of the article (90). The elastic laminate (10) can also be used as the side flaps (94, 95). The elastic laminate (10) having only one fabric layer (20) wherein the fabric layer (20) is the body facing surface of the side flaps (92) and the elastic layer (30) is the garment facing surface of side flaps (92) can be preferable as the garment facing surface of the side flaps (92) does not touch skin of wearer. The elastic laminate (10) can be used in a rectangular shape and then cut to have the shape of the side flaps (94, 95) in manufacturing. One of the functional zones (23, 24, 43, 44) of the laminate (10) coincides with the side edge of the side flap (94, 95) which is connected to the main body (91) while the other one coincides with the side edge of the side flap (94, 95) which is far from the main body (91). The one which is close to the main body (91) contributes to obtain adequate bonding ability and tear strength for the junction areas in which the side edges of the side flaps (94, 95) are connected to the main body (91). The other one which is far from the main body (91) serves for a force application area for a wearer to fix the article (90) to her garment. Rigidity of the functional zone (23, 24, 43, 44) in this area restricts application of force and prevents over-elongation. So, elongation and retraction of the side flaps (94, 95) can be maintained at a desired level hindering deformation and favoring preservation of their shape. However, the elastic laminate (10) having only one functional zone (23, 43) can also be used as the side flaps (94, 95) rather than the one having two functional zones (23, 24, 43, 44). Sealed area (96) is formed by joining the body facing layer (92) and garment facing layer (93) by application of adhesive, thermal and mechanical forces. The surface pattern of the sealed area (96) is different from the rest of the surface of the body facing layer (92) and garment facing layer (93). In sealed area (96) a slight fusion of polymeric layers occurs due to applied heat and pressure which provides interference of the layers (92, 93) and fairly higher bonding strength compared to use of adhesive only. Therefore, it cannot be so necessary to use the elastic laminate (10) having two functional zones (23, 24, 43, 44) to enhance bonding ability at the sealed area (96). Use of the laminate (10) with only one functional zone (23, 43) overlapping the sealed area (96) can be functional enough to ensure required bonding ability at the sealed area (96) due to the positive effect of application of heat and pressure on bonding.

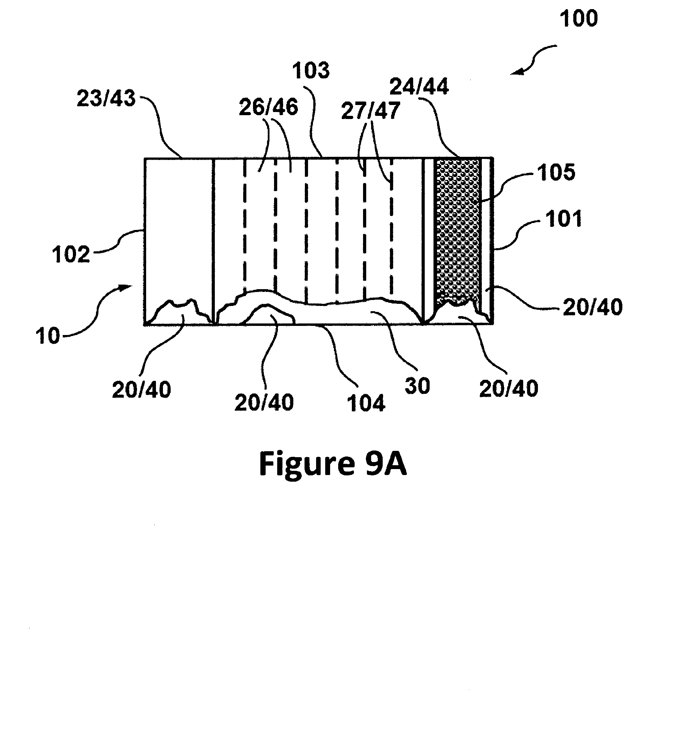

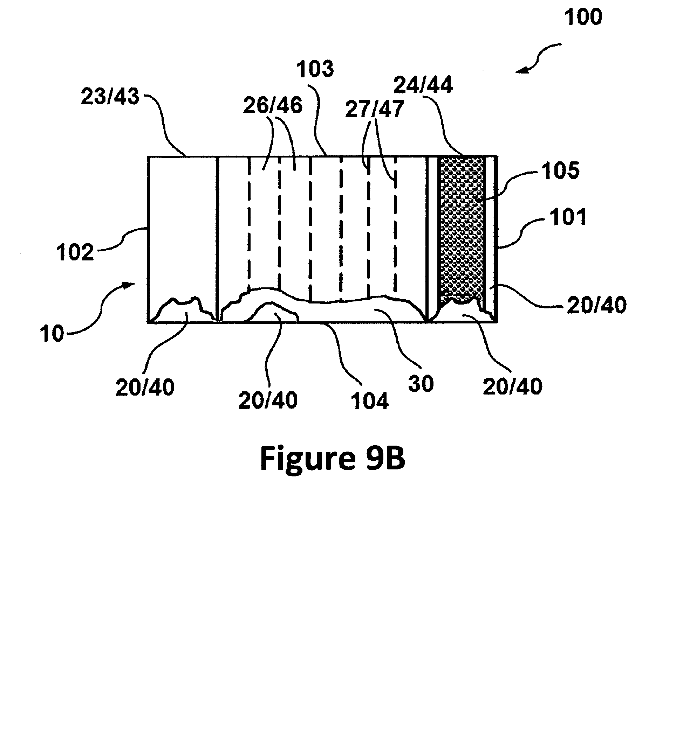

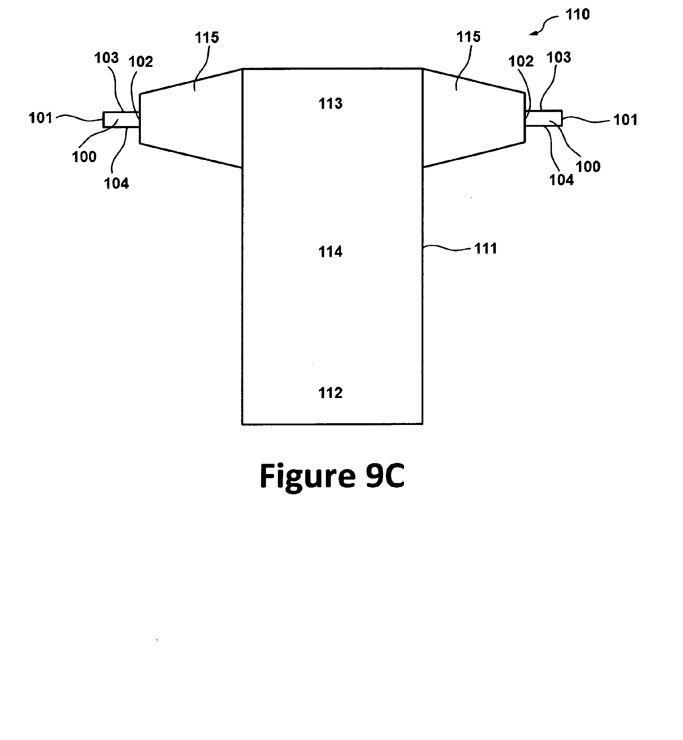

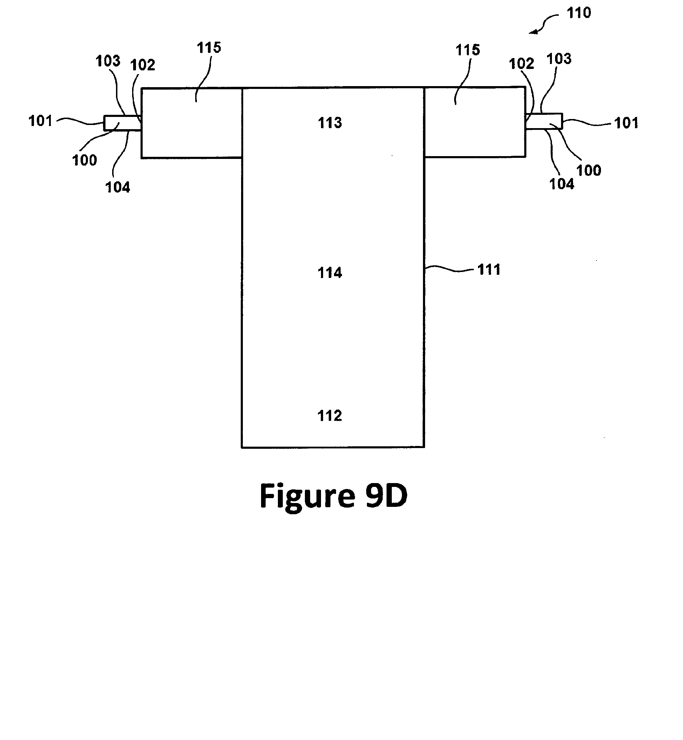

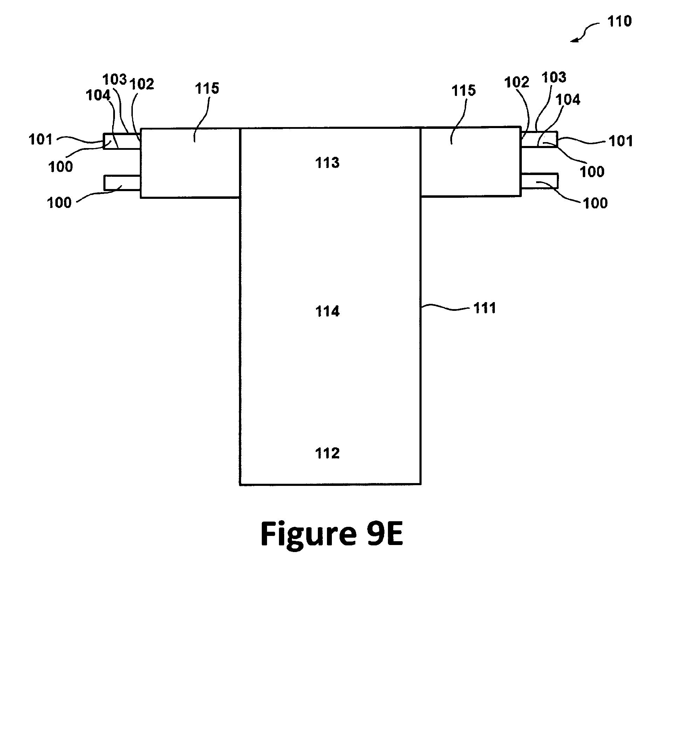

The elastic laminate (10) can be used to make a fastening tape (100) (Figures 9A and 9B) of an absorbent article (110) (Figures 9C-9E) which comprise a chassis (111) including a front region (112), back region (113), a crotch region (114) between the front (112) and back region (113) and rear side panels (115). The rear side panel (115) may or may not include the elastic laminate (10). The fastening tape (100) can comprise a terminal edge (101), a neighboring edge (102), an upper edge (103), a lower edge (104) and a fixing element (105) (e.g., hooks/loops) used for donning the article (110) to wearer's body. The lateral edge of the side panel (115) is connected to the neighboring edge (102) of the fastening tape (100). The first fabric layer (20) or the second fabric layer (40) can be the skin facing side of the fastening tape (100) or the first fabric layer (20) or the second fabric layer (40) can be the outermost surface of the fastening tape (100). The distance between the two edges of the laminate (10) in the cross direction (C) coincide with the distance between the terminal edge (101) and neighboring edge (102) of the fastening tape (100). The machine direction (M) of the laminate (10) coincides with the distance between the upper (103) and lower edge (104). The fastening tape (100) is extensible in the direction of neighboring-terminal edge (102-101). Two fastening tapes (100) can be used on rectangular shaped side panels (115) (Figure 9E). One of the functional zones (23, 24, 43, 44) of the laminate (10) coincides with the terminal edge (101) and the other one coincides with the neighboring edge (102). The one which coincides with the neighboring edge (102) contributes to obtain adequate bonding ability and tear strength for the junction areas in which the neighboring edges (102) are connected to the side panels (115). The other functional zone (23, 24, 43, 44) which coincides with the terminal edge (101) serves for an application area for attaching an additional component such as the fixing element (105). Similarly, this functional zone (23, 24, 43, 44) contributes to obtain adequate bonding ability and tear strength for the junction areas in which the fixing elements (105) are connected to the terminal edges (101). Additionally, rigidity of the functional zone (23, 24, 43, 44) in this area restricts application of force and prevents over-elongation. So, elongation and retraction of fastening tapes (100) can be maintained at a desired level hindering deformation and favoring preservation of their shape.

After the detailed descriptions above it can be concluded that the elastic laminates (10) of the present invention are promising alternative components to be used in several regions of absorbent hygienic articles. The design of the elastic laminate (10) can be diversified in such a way that it can be applied to various nonwoven materials in different ways. In addition, diversification of the design provides obtaining elastic laminates with adjustable elastic properties using the same equipment in a simple and economical manner.

### TEST METHODS

### A: Zwick/Roell 10N Test Method:

In this test method elongation (%) of the elastic laminate (10) samples of the present invention was measured by Zwick/Roell instrument applying a force of 10N on 40mm width of the samples. The test procedure is originated from ASTM D882 standard.

Laminates (10) to be tested are cut into pieces having 40 mm of width which is the direction parallel to the rows of slits (M). The test parameters to be adjusted in software are given in Table 1.

**TABLE 1. Test Parameters of Zwick/Roell 10N Test Method**

| **Test Parameter** | **Numerical Value** |
|---|---|
| Test speed | 500 mm/min |
| Preload speed | 50 mm/min |
| Preload | 0,04 N |
| Target position | Standard force, 10 N |
| Wait time | 60 seconds |
| Type of wait | Force controlled |
| Number of iterations | 4 |

For performing the tests the sample is placed between the upper and lower clamps in the direction perpendicular to the rows of slits (C). The upper edge of the sample is placed in the upper clamp and pneumatic valve connected to the upper clamp is closed to shut the clamp and nip the upper edge of the sample which is parallel to rows of slits (M) and of 40mm width. The force is set to zero on the screen of software. The lower edge of the sample is placed in the lower clamp and pneumatic valve connected to the lower clamp is closed to shut the clamp and nip the lower edge of the sample which is parallel to rows of slits (M) and of 40mm width. The test is started using the instruction button on the screen of software.

The sample is pulled in the upward direction by the upper clamp of the instrument while the lower clamp is immobile. The sample is pulled until the tensile force reaches to 10 N. The force is released after the sample is kept at elongated state under the tensile force of 10 N for 1 minute. After that upper clamp releases automatically and turns back to initial position. The value of "S after hold 1, %" appeared on the screen of the software is recorded.

### B: Test Method for Determination of Basis Weight:

In this test method basis weight of the elastic laminate (10), fabric layer (20, 40) and elastic layer (30) is determined. Samples to be tested are cut into pieces which have a width of 10 cm and a length of 10 cm. 4 samples are prepared in this way and each sample is weighed. The weight of each sample is divided to surface area of the sample to find the basis weight of the sample.

### EXAMPLES and TESTS

### EXAMPLE 1. Tests and Calculations for The Elastic Laminate Having 1 Fabric Layer, 1 Elastic Layer and 1 Functional Zone

As also mentioned before in the text the elastic laminate (10) of the present invention can be used as the rear side panel (70), garment facing layer (86) and side flaps (94, 95) of the absorbent articles. The percentage ratios of the width of the functional zone (23) and width of the activated zone (25) to the width of the elastic laminate (10) were calculated for each component in case of having only one fabric layer (20) and functional zone (23). The results are given below in Table 2. It can be concluded that the width of the functional zone (23) varies between 4,17% - 53,33% while the width of activated zone (25) is in the range of 46,67% - 95,83% regarding the all components.

**TABLE 2. The Percentage Ratios of The Width of The Functional Zone and Width of The Activated Zone to The Width of The Elastic Laminate for The Components of The Absorbent Articles (1 Fabric Layer, 1 Elastic Layer and 1 Functional Zone)**

| **COMPONENT** | **Width of the functional zone (mm)** | | **Width of the activated zone (mm)** | | **Width of the functional zone (%)** | | **Width of the activated zone (%)** | |
|---|---|---|---|---|---|---|---|---|
| | **MIN** | **MAX** | **MIN** | **MAX** | **MIN** | **MAX** | **MIN** | **MAX** |
| **Rear side panel*** | 10 | 20 | 45 | 55 | 15,38% | 30,77% | 69,23% | 84,62% |
| | 10 | 20 | 55 | 65 | 13,33% | 26,67% | 73,33% | 86,67% |
| | 10 | 20 | 60 | 70 | 12,50% | 25,00% | 75,00% | 87,50% |
| | 10 | 20 | 80 | 90 | 10,00% | 20,00% | 80,00% | 90,00% |
| **Body facing layer*** | 10 | 20 | 180 | 190 | 5,00% | 10,00% | 90,00% | 95,00% |
| | 10 | 20 | 190 | 200 | 4,76% | 9,52% | 90,48% | 95,24% |
| | 10 | 20 | 200 | 210 | 4,55% | 9,09% | 90,91% | 95,45% |
| | 10 | 20 | 210 | 220 | 4,35% | 8,70% | 91,30% | 95,65% |
| | 10 | 20 | 220 | 230 | 4,17% | 8,33% | 91,67% | 95,83% |
| **Side flaps*** | 10 | 20 | 17,5 | 27,5 | 26,67% | 53,33% | 46,67% | 73,33% |
| | 10 | 20 | 20 | 30 | 25,00% | 50,00% | 50,00% | 75,00% |
| **Fastening tape*** | 10 | 20 | 30 | 40 | 60,00% | 80,00% | 20,00% | 40,00% |
| *The number of the rows for each component refers to the components of different sizes constituted from different sizes of absorbent articles. | | | | | | | | |

Zwick/Roell 10N test was performed to measure the elongation of the activated zone (25) of the said elastic laminate (10). The elastic laminate (10) was manufactured using 35 g/m² elastic layer (30) having a width of 100 mm and 20 g/m² spunbond nonwoven layer having the width of 100 mm as the first fabric layer (20). The layers (20, 30) were laminated using 10 g/m² adhesive. The resulting laminate (10) has a basis weight in the range of 65-70 g/m² and a width of 100 mm. The test results given in Table 3 show that the elastic laminate (10) having only one fabric layer (20) one elastic layer (30) one activated zone(25) and one functional zone (23) can elongate in the range of 57,75% - 81,25% of its width in relaxed state. Not being limited to the present examples the two layered elastic laminates activated zones of the present invention can elongate in the range of 50% -%150.

**TABLE 3. Zwick/Roell 10N Test Results of The Elastic Laminate**

| | **Before test** | **After test** | **Elongation** |
|---|---|---|---|
| **ELASTIC LAMINATE 1 fabric layer** | **Width of the activated zone (mm)** | **Width of the activated zone (mm)** | |
| **1 elastic layer** | 40,00 | 63,10 | 57,75% |
| **1 functional zone** | 40,00 | 66,02 | 65,05% |
| | 40,00 | 68,20 | 70,50% |
| | 40,00 | 72,50 | 81,25% |
| **Average** | 40,00 | 67,46 | 68,64% |
| **Maximum** | 40,00 | 72,50 | 81,25% |
| **Minimum** | 40,00 | 63,10 | 57,75% |
| **Standard Deviation** | 0,00 | 3,96 | 9,90% |

### EXAMPLE 2. Tests and Calculations for The Elastic Laminate Having 1 Fabric Layer, 1 Elastic Layer and 2 Functional Zones

The percentage ratios of the total width of the functional zones (23, 24) and width of the activated zone (25) to the width of the elastic laminate (10) were calculated for each component in case of having only one fabric layer (20) and two functional zones (23, 24). The results are given below in Table 4. It can be concluded that the total width of the functional zones (23, 24) varies between 8,33% - 80,00% while the width of activated zone (25) is in the range of 20,00% - 91,67% regarding the all components. Said laminate elongates in the same range with the one in Example 1 (see Table 3) as the width of the activated zone which the test is applied to has the same value with the one in Example 1.

**TABLE 4. The Percentage Ratios of The Total Width of the Functional Zones and Width of the Activated Zone to the Width of The Elastic Laminate for the components of absorbent articles (1 fabric layer, 1 elastic layer and 2 functional zones)**

| **COMPONENT** | **Width of the functional zone (mm)** | | **Width of the activated zone (mm)** | | **Width of the functional zone (%)** | | **Width of the activated zone (%)** | |
|---|---|---|---|---|---|---|---|---|
| | **MIN** | **MAX** | **MIN** | **MAX** | **MIN** | **MAX** | **MIN** | **MAX** |
| **Rear side panel*** | 20 | 40 | 25 | 45 | 30,77% | 61,54% | 38,46% | 69,23% |
| | 20 | 40 | 35 | 55 | 26,67% | 53,33% | 46,67% | 73,33% |
| | 20 | 40 | 40 | 60 | 25,00% | 50,00% | 50,00% | 75,00% |
| | 20 | 40 | 60 | 80 | 20,00% | 40,00% | 60,00% | 80,00% |
| **Body facing layer*** | 20 | 40 | 160 | 180 | 10,00% | 20,00% | 80,00% | 90,00% |
| | 20 | 40 | 170 | 190 | 9,52% | 19,05% | 80,95% | 90,48% |
| | 20 | 40 | 180 | 200 | 9,09% | 18,18% | 81,82% | 90,91% |
| | 20 | 40 | 190 | 210 | 8,70% | 17,39% | 82,61% | 91,30% |
| | 20 | 40 | 200 | 220 | 8,33% | 16,67% | 83,33% | 91,67% |
| **Side flaps*** | 20 | 40 | - | 20 | 50,00% | - | - | 50,00% |
| **Fastening tape*** | 20 | 40 | 10 | 30 | 40,00% | 80,00% | 20,00% | 60,00% |
| *The number of the rows for each component refers to the components of different sizes for different sizes of absorbent articles. | | | | | | | | |

### EXAMPLE 3. Tests for The Elastic Laminate Having 2 Fabric Layers, 1 Elastic Layer and 2 Functional Zones

The elastic laminate having 2 fabric layers and 1 elastic film and 2 functional zones has the same percentage ratios of the total width of the functional zones (23, 24, 43, 44) and width of the activated zone (25, 45) to the width of the elastic laminate (10) with the one having 1 fabric layer and 2 functional zones given in Example 2 (see Table 4) as the first and second fabric layers are symmetrical.

Zwick/Roell 10N test was performed to measure the elongation of the activated zone (25, 45) of the elastic laminate (10) having two fabric layers (20, 40) one elastic layer (30) activated zones (25) and functional zones (23, 24, 43, 44). The elastic laminate was (10) manufactured using 35 g/m² elastic layer having a width of 100 mm and 20 g/m² spunbond nonwoven layers having the width of 100 mm as the first fabric layer (20) and the second fabric layer (40). The layers were laminated using 10 g/m² adhesive for each fabric layer (20, 40). The resulting laminate (10) has a basis weight in the range of 100-110 g/m² and a width of 100 mm. The test results given in Table 5 show that the elastic laminate (10) having two fabric layers (20, 40) one elastic layer (30) activated zones (25) and functional zones (23, 24, 43, 44) can elongate in the range of 35,50% - 61,83% of its width in relaxed state (See Table 5). Not being limited to the present examples the three layered elastic laminates activated zones of the present invention can elongate in the range of 25% -%150.

**TABLE 5. Zwick/Roell 10N Test Results of The Elastic Laminate**

| | **Before test** | **After test** | **Elongation** |
|---|---|---|---|
| **ELASTIC LAMINATE 2 fabric layers** | **Width of the activated zone (mm)** | **Width of the activated zone (mm)** | |
| **1 elastic layer** | 40,00 | 58,59 | 46,48% |
| **2 functional zones** | 40,00 | 64,73 | 61,83% |
| | 40,00 | 54,20 | 35,50% |
| | 40,00 | 55,15 | 37,88% |
| **Average** | 40,00 | 58,17 | 45,42% |
| **Maximum** | 40,00 | 64,73 | 61,83% |
| **Minimum** | 40,00 | 54,20 | 35,50% |
| **Standard Deviation** | 0,00 | 4,76 | 11,91% |

## Claims

1. An elastic laminate (10) comprising an elastic layer (30) having a first surface and a second surface, and a first nonwoven layer (20) bonded to said first surface of said elastic layer, said elastic laminate having at least one primary direction of stretch (C)
**characterized in that**,
said nonwoven layer (20) has an activated zone (25), said first activated zone having first and second longitudinal edges and a width and a functional zone (23),
said functional zone (23) being positioned adjacent said first longitudinal edge of said activated zone, said activated zone having a plurality of openings in rows (271) and axis of stretch (C) which is essentially perpendicular to direction of opening rows (M), said elastic laminate having a closed width when relaxed and an open width by application of a force, wherein said openings are slits arranged in a brick-like pattern of rows.

2. An elastic laminate according to claim 1, wherein
said functional zone has a width that is in the range of 5% to 80% of the width of said elastic laminate and
said activated zone having a width in the range of 20% to 95% of the width of said elastic laminate and
when a force of 2.5 N/cm applied to said elastic laminate along said axis of stretch said activated zone undergoes a stretch in direction of axis of stretch in the range of 50% to 150% whereas stretch is calculated as the difference of open width of activated zone and closed width of activated zone divided to closed width of activated zone in percentage,
whereby applied force has to be determined according to the test method performed using Zwick/Roell 10N test method as described in the specification.

3. The elastic laminate of claim 2, wherein
said slits are 5 mm to 40 mm of length and
said slits within each of said rows are spaced 1 mm to 30 mm apart and
said rows are spaced 1 mm to 30 mm apart.

4. The elastic laminate of claim 3, wherein
said slits are 12 mm long and
said slits within each of said rows are spaced 1 mm apart and
said rows are spaced 2 mm apart.

5. An elastic laminate according to claim 2, wherein
the width of functional zone is in the range of 10% to 40% of the width of the elastic laminate and
the width of activated zone is in the range of 60% to 90% of the width of elastic laminate.

6. The elastic laminate of claim 5, wherein
said slits are 5 mm to 20 mm of length and
said slits within each of said rows are spaced 1 mm to 5 mm apart and
said rows are spaced 1 mm to 5 mm apart.

7. The elastic laminate of claim claim 6, wherein
said slits are 12 mm long and
said slits within each of said rows are spaced 1 mm apart and
said rows are spaced 2 mm apart.

8. The elastic laminate of claim 1, further comprising a second functional zone (24), said second functional zone (24) being positioned adjacent said second longitudinal side of said activated zone (25).

9. An elastic laminate according to claim 8, wherein
said first and said second functional zones having a combined width that is in the range of 5% to 80% of the width of said elastic laminate and
activated zone having a width in the range of 20% to 95% of the width of said elastic laminate and
when a force of 2.5 N/cm is applied to said elastic laminate along said axis of stretch said activated zone undergoes a stretch in direction of axis of stretch in the range of 50% to 150%.
whereas stretch is calculated as the difference of open width of activated zone and closed width of activated zone divided to closed width of activated zone in percentage,
whereby applied force has to be determined according to the test method performed using Zwick/Roell 10N test method as described in the specification.

10. An elastic laminate according to claim 9, wherein
said slits are 5 mm to 40 mm of length and
said slits within each of said rows are spaced 1 mm to 30 mm apart and
said rows are spaced 1 mm to 30 mm apart.

11. An elastic laminate according to claim 10, wherein
said slits are 12 mm long and
said slits within each of said rows are spaced 1 mm apart and
said rows are spaced 2mm apart.

12. An elastic laminate according to claim 9, wherein
said first and said second functional zones having a combined width that is in the range of 10% to 65% of the width of said elastic laminate and
an activated zone having a width in the range of 35% to 85% of the width of said elastic laminate.

13. An elastic laminate according to claim 12, wherein
said slits are 5 mm to 20 mm of length and
said slits within each of said rows are spaced 1 mm to 5 mm apart and
said rows are spaced 1 mm to 5 mm apart.

14. An elastic laminate according to claim 13, wherein
said slits are 12 mm long and
said slits within each of said rows are spaced 1 mm apart and
said rows are spaced 2mm apart.

15. An elastic laminate according to claim 9, wherein
said first and said second functional zones having a combined width that is in the range of 5% to 35% of the width of said elastic laminate and
an activated zone having a width in the range of 65% to 95% of the width of said elastic laminate and

16. An elastic laminate according to claim 15, wherein
said slits are 5 mm to 20 mm of length and
said slits within each of said rows are spaced 1 mm to 5 mm apart and
said rows are spaced 1 mm to 5 mm apart.

17. An elastic laminate according to claim 16, wherein
said slits are 12 mm long and
said slits within each of said rows are spaced 1 mm apart and
said rows are spaced 2mm apart.

18. The elastic laminate of claim 8 further comprising a second activated zone and a third functional zone, said third functional zone being positioned between said first and said second activated zones, said second activated zone being positioned between said first functional zone and said third functional zone.

19. The elastic laminate of claim 1, claim 8 and claim 18 further comprising a second nonwoven layer (40) bonded to said second surface of said elastic laminate (30).

20. An elastic laminate according to claim 19, wherein
said second nonwoven layer (40) comprise one or a plurality of activated zones (45) and one or a plurality of functional zones (44) having the same stretch direction (C) of the first nonwoven layer (20).

21. An elastic laminate according to claim 20, wherein
said functional zones having a combined width that is in the range of 5% to 65% of the width of said elastic laminate and
said activated zones having a combined width in the range of 35% to 95% of the width of said elastic laminate and
when a force of 2.5 N/cm is applied to said elastic laminate along said axis of stretch said activated zone undergoes a stretch in direction of axis of stretch in the range of 25% to 150 %,
whereas stretch is calculated as the difference of open width of activated zone and closed width of activated zone divided to closed width of activated zone in percentage,
whereby applied force has to be determined according to the test method performed using Zwick/Roell 10N test method as described in the specification.

22. An elastic laminate according to claim 21, wherein
said slits are 5 mm to 40 mm of length and
said slits within each of said rows are spaced 1 mm to 30 mm apart and
said rows are spaced 1 mm to 30 mm apart.

23. An elastic laminate according to claim 22, wherein
said slits are 12 mm long and
said slits within each of said rows are spaced 1 mm apart and
said rows are spaced 2mm apart.

24. An elastic laminate according to claim 19, wherein
said second nonwoven layer is comprising of a functional zone having characteristic feature of being expandable.

25. An elastic laminate according to claim 19, wherein
said first nonwoven layer (20) and second nonwoven layer (40) are comprising one or a plurality of activated zones and one or a plurality of functional zones and activated zones are adjacent to functional zones and first and second nonwoven layers' activated zones are optionally not located vis a vis.

26. An elastic laminate according to claim 25, wherein
elastic layer covers less than 90% of the width of elastic laminate.

27. An elastic laminate according to claim 26, wherein
elastic layer additionally does not cover 5% to 80% of the width of the middle part of elastic laminate in the stretch direction.

28. The elastic laminate according to preceding claims, wherein functional zones are capable of being bonded to other materials.

29. The elastic laminate according to preceding claims, wherein functional zones facilitate the further processing of said elastic laminate.

30. The elastic laminate according to preceding claims, wherein functional zones are capable of accepting force application without deformation and tearing, applied from sides of wearer, mother or caregiver.

31. The elastic laminate according to preceding claims, wherein functional zones are optionally printed.

32. Process for the preparation of elastic laminates according to preceding claims comprising
i: open cutting slits to a nonwoven layer,
ii: joining an elastic layer onto surface of nonwowen layer,
iii: joining optionally a nonwowen layer which is optionally slitted, to the other side of elastic layer,
iv: combining the layers together under adjoining application
wherein for combining said layers optionally glueing, heating and ultrasonic methods are applied before or during adjoining application.

33. Use of elastic laminates of preceding claims in preparation of wearable articles.

34. Use of elastic laminates of preceding claims in preparation of disposable absorbent article parts comprising of elastic ears and or panels, backsheet, fastening tape, waist parts of baby diapers, adult diapers, incontinence diapers, pant like diapers and side flaps of feminine pads and incontinence pads.

## Amended claims

### Amended claims in accordance with Rule 137(2) EPC.

1. An elastic laminate (10) for use in disposable absorbent article parts, comprising an elastic layer (30) having a first surface and a second surface, and a first nonwoven layer (20) bonded to said first surface of said elastic layer, said elastic laminate having at least one primary direction of stretch (C) wherein,
said nonwoven layer (20) has an activated zone (25), said first activated zone having first and second longitudinal edges and a width and a functional zone (23),
said functional zone (23) being positioned adjacent said first longitudinal edge of said activated zone, said activated zone having a plurality of openings in rows (271) and axis of stretch (C) which is essentially perpendicular to direction of opening rows (M), said elastic laminate having a closed width when relaxed and an open width by application of a force, wherein said openings are slits arranged in a brick-like pattern of rows,
**characterized in that**,
said functional zones are capable of being bonded to other materials,
and capable of accepting force application without deformation and tearing, applied from sides of wearer, mother or caregiver,
and facilitate the further processing.

2. An elastic laminate according to claim 1, wherein
said functional zone has a width that is in the range of 5% to 80% of the width of said elastic laminate and
said activated zone having a width in the range of 20% to 95% of the width of said elastic laminate and
when a force of 2.5 N/cm applied to said elastic laminate along said axis of stretch said activated zone undergoes a stretch in direction of axis of stretch in the range of 50% to 150%
whereas stretch is calculated as the difference of open width of activated zone and closed width of activated zone divided to closed width of activated zone in percentage,
whereby applied force has to be determined according to the test method performed using Zwick/Roell 10N test method as described in the specification.

3. The elastic laminate of claim 2, wherein
said slits are 5 mm to 40 mm of length and
said slits within each of said rows are spaced 1 mm to 30 mm apart and
said rows are spaced 1 mm to 30 mm apart.

4. The elastic laminate of claim 3, wherein
said slits are 12 mm long and
said slits within each of said rows are spaced 1 mm apart and
said rows are spaced 2 mm apart.

5. An elastic laminate (10) according to preceding claims for use in disposable absorbent article parts, wherein
said disposable absorbent article parts are selected from rear side panel, backsheet or fastening tape parts of baby diapers, adult diapers, incontinence diapers or side flaps of feminine pads and incontinence pads.

6. A rear side panel according to claim 5, wherein
the width of functional zone is in the range of 10% to 40% of the width of the elastic laminate and the width of activated zone is in the range of 60% to 90% of the width of elastic laminate.

7. A rear side panel according to claim 6, wherein
said rear side panel further comprising a second functional zone (24),
said second functional zone (24) being positioned adjacent said second longitudinal side of said activated zone (25).

8. A rear side panel according to claim 7, wherein
said first and said second functional zones having a combined width that is in the range of 10% to 65% of the width of said elastic laminate and
an activated zone having a width in the range of 35% to 85% of the width of said elastic laminate.

9. The elastic laminate of claim 1 and claim 7 further comprising a second nonwoven layer (40) bonded to said second surface of said elastic laminate (30).

10. An elastic laminate according to claim 9, wherein
said second nonwoven layer (40) comprise one or a plurality of activated zones (45) and one or a plurality of functional zones (44) having the same stretch direction (C) of the first nonwoven layer (20).

11. Process for the preparation of elastic laminates according to preceding claims comprising
i: open cutting slits to a nonwoven layer,
ii: joining an elastic layer onto surface of nonwowen layer,
iii: joining optionally a nonwowen layer which is optionally slitted, to the other side of elastic layer,
iv: combining the layers together under adjoining application
wherein for combining said layers optionally glueing, heating and ultrasonic methods are applied before or during adjoining application.
